(19) Europäisches Patentamt
European Patent Office
Office européen des brevets

(11) **EP 1 196 603 B1**

(12) **EUROPÄISCHE PATENTSCHRIFT**

(45) Veröffentlichungstag und Bekanntmachung des Hinweises auf die Patenterteilung:
**22.08.2007 Patentblatt 2007/34**

(21) Anmeldenummer: **00951461.3**

(22) Anmeldetag: **27.07.2000**

(51) Int Cl.:
*C12N 15/53* (2006.01)       *C12N 15/70* (2006.01)
*C12N 9/02* (2006.01)        *C12N 1/21* (2006.01)
*C12P 7/42* (2006.01)

(86) Internationale Anmeldenummer:
**PCT/EP2000/007252**

(87) Internationale Veröffentlichungsnummer:
**WO 2001/007574 (01.02.2001 Gazette 2001/05)**

(54) **MODIFIZIERTE CYTOCHROM P450-MONOOXYGENASEN**

MODIFIED CYTOCHROME P450 MONOOXYGENASES

MONOOXYGENASES A CYTOCHROME P450 MODIFIEES

(84) Benannte Vertragsstaaten:
**AT BE CH CY DE DK ES FI FR GB GR IE IT LI LU MC NL PT SE**

(30) Priorität: **27.07.1999  DE 19935115**
**10.03.2000  DE 10011723**

(43) Veröffentlichungstag der Anmeldung:
**17.04.2002  Patentblatt 2002/16**

(73) Patentinhaber: **BASF Aktiengesellschaft**
**67056 Ludwigshafen (DE)**

(72) Erfinder:
• **HAUER, Bernhard**
**D-67056 Fussgönheim (DE)**
• **PLEISS, Juergen**
**D-71679 Asperg (DE)**
• **SCHWANEBERG, Ulrich**
**D-71336 Waiblingen (DE)**
• **SCHMITT, Jutta**
**D-70563 Stuttgart (DE)**
• **FISCHER, Markus**
**D-71638 Ludwigsburg (DE)**
• **SCHMID, Rolf**
**D-70329 Stuttgart (DE)**
• **LI, Qing-shan**
**Kyoto 606-8502 (JP)**

(74) Vertreter: **Kinzebach, Werner et al**
**Ludwigsplatz 4**
**67059 Ludwigshafen (DE)**

(56) Entgegenhaltungen:
**GB-A- 2 294 692**

• OLIVER C F ET AL: "A single mutation in cytochrome P450 BM3 changes substrate orientation in a catalytic intermediate and the regiospecificity of hydroxylation" BIOCHEMISTRY, AMERICAN CHEMICAL SOCIETY. EASTON, PA, US, Bd. 36, 1997, Seiten 1567-1572, XP002131694 ISSN: 0006-2960
• CHEN JIAN-KANG ET AL: "Transfection of an active cytochrome P450 arachidonic acid epoxygenase indicates that 14,15-epoxyeicosatrienoic acid functions as an intracellular second messenger in response to epidermal growth factor" JOURNAL OF BIOLOGICAL CHEMISTRY, Bd. 274, Nr. 8, 19. Februar 1999 (1999-02-19), Seiten 4764-4769, XP002345066 ISSN: 0021-9258
• ALWORTH W L ET AL: "A site-specific mutant of the bacterial cytochrome P450 102 (BM-3) possessing a new capability to catalyze the hydroxylation of the polycyclic aromatic hydrocarbons pyrene and benzo(a)pyrene" FASEB JOURNAL, Bd. 9, Nr. 6, 1995, Seite A1491, XP008053167 & ANNUAL MEETING OF THE AMERICAN SOCIETY FOR BIOCHEMISTRY AND MOLECULAR BIOLOGY; SAN FRANCISCO, CALIFORNIA, USA; MAY 21-25, 1995 ISSN: 0892-6638
• NOBLE M A ET AL: "ROLES OF KEY ACTIVE-SITE RESIDUES IN FLAVOCYTROCHROME P450 BM3" BIOCHEMICAL JOURNAL, PORTLAND PRESS, LONDON, GB, Bd. 339, April 1999 (1999-04), Seiten 371-379, XP001011145 ISSN: 0264-6021

- LI Q-S ET AL: "Rational evolution of a medium chain-specific cytochrome P-450 BM-3 variant" BIOCHIMICA ET BIOPHYSICA ACTA. PROTEIN STRUCTURE AND MOLECULAR ENZYMOLOGY, ELSEVIER, AMSTERDAM,, NL, Bd. 1545, Nr. 1-2, 9. Februar 2001 (2001-02-09), Seiten 114-121, XP004248836 ISSN: 0167-4838

- LENTZ OLIVER ET AL: "Modification of the fatty acid specificity of cytochrome P450 BM-3 from Bacillus megaterium by directed evolution: A validated assay" JOURNAL OF MOLECULAR CATALYSIS B ENZYMATIC, Bd. 15, Nr. 4-6, 1. November 2001 (2001-11-01), Seiten 123-133, XP002346677 ISSN: 1381-1177

SEQUENZPROTOROLL

<110> BASF Aktiengesellschaft
<120> Modifizierte Cytochrom P450 Monoxygenasen
<130> M/40434
<140>
<141>
<160> 20
<170> PatentIn Ver. 2.1
<210> 1
<211> 3150
<212> DNA
<213> Bacillus megaterium
<220>
<221> CDS
<222> (4)..(3150)
<400> 1

```
atg aca att aaa gaa atg cct cag cca aaa acg ttt gga gag ctt aaa     48
    Thr Ile Lys Glu Met Pro Gln Pro Lys Thr Phe Gly Glu Leu Lys
     1               5                  10                  15

aat tta ccg tta tta aac aca gat aaa ccg gtt caa gct ttg atg aaa     96
Asn Leu Pro Leu Leu Asn Thr Asp Lys Pro Val Gln Ala Leu Met Lys
                 20                  25                  30

att gcg gat gaa tta gga gaa atc ttt aaa ttc gag gcg cct ggt cgt    144
Ile Ala Asp Glu Leu Gly Glu Ile Phe Lys Phe Glu Ala Pro Gly Arg
                     35                  40                  45

gta acg cgc tac tta tca agt cag cgt cta att aaa gaa gca tgc gat    192
Val Thr Arg Tyr Leu Ser Ser Gln Arg Leu Ile Lys Glu Ala Cys Asp
                 50                  55                  60

gaa tca cgc ttt gat aaa aac tta agt caa gcg ctt aaa ttt gta cgt    240
Glu Ser Arg Phe Asp Lys Asn Leu Ser Gln Ala Leu Lys Phe Val Arg
             65                  70                  75
```

```
gat ttt gca gga gac ggg tta ttt aca agc tgg acg cat gaa aaa aat    288
Asp Phe Ala Gly Asp Gly Leu Phe Thr Ser Trp Thr His Glu Lys Asn
    80              85              90              95

tgg aaa aaa gcg cat aat atc tta ctt cca agc ttc agt cag cag gca    336
Trp Lys Lys Ala His Asn Ile Leu Leu Pro Ser Phe Ser Gln Gln Ala
                100             105             110

atg aaa ggc tat cat gcg atg atg gtc gat atc gcc gtg cag ctt gtt    384
Met Lys Gly Tyr His Ala Met Met Val Asp Ile Ala Val Gln Leu Val
                115             120             125

caa aag tgg gag cgt cta aat gca gat gag cat att gaa gta ccg gaa    432
Gln Lys Trp Glu Arg Leu Asn Ala Asp Glu His Ile Glu Val Pro Glu
                130             135             140

gac atg aca cgt tta acg ctt gat aca att ggt ctt tgc ggc ttt aac    480
Asp Met Thr Arg Leu Thr Leu Asp Thr Ile Gly Leu Cys Gly Phe Asn
                145             150             155

tat cgc ttt aac agc ttt tac cga gat cag cct cat cca ttt att aca    528
Tyr Arg Phe Asn Ser Phe Tyr Arg Asp Gln Pro His Pro Phe Ile Thr
    160             165             170             175

agt atg gtc cgt gca ctg gat gaa gca atg aac aag ctg cag cga gca    576
Ser Met Val Arg Ala Leu Asp Glu Ala Met Asn Lys Leu Gln Arg Ala
                180             185             190

aat cca gac gac cca gct tat gat gaa aac aag cgc cag ttt caa gaa    624
Asn Pro Asp Asp Pro Ala Tyr Asp Glu Asn Lys Arg Gln Phe Gln Glu
                195             200             205

gat atc aag gtg atg aac gac cta gta gat aaa att att gca gat cgc    672
Asp Ile Lys Val Met Asn Asp Leu Val Asp Lys Ile Ile Ala Asp Arg
                210             215             220

aaa gca agc ggt gaa caa agc gat gat tta tta acg cat atg cta aac    720
Lys Ala Ser Gly Glu Gln Ser Asp Asp Leu Leu Thr His Met Leu Asn
                225             230             235

gga aaa gat cca gaa acg ggt gag ccg ctt gat gac gag aac att cgc    768
```

```
Gly Lys Asp Pro Glu Thr Gly Glu Pro Leu Asp Asp Glu Asn Ile Arg
240             245             250             255

tat caa att att aca ttc tta att gcg gga cac gaa aca aca agt ggt     816
Tyr Gln Ile Ile Thr Phe Leu Ile Ala Gly His Glu Thr Thr Ser Gly
                260             265             270

ctt tta tca ttt gcg ctg tat ttc tta gtg aaa aat cca cat gta tta   864
Leu Leu Ser Phe Ala Leu Tyr Phe Leu Val Lys Asn Pro His Val Leu
                275             280             285

caa aaa gca gca gaa gaa gca gca cga gtt cta gta gat cct gtt cca   912
Gln Lys Ala Ala Glu Glu Ala Ala Arg Val Leu Val Asp Pro Val Pro
                290             295             300

agc tac aaa caa gtc aaa cag ctt aaa tat gtc ggc atg gtc tta aac   960
Ser Tyr Lys Gln Val Lys Gln Leu Lys Tyr Val Gly Met Val Leu Asn
                305             310             315

gaa gcg ctg cgc tta tgg cca act gct cct gcg ttt tcc cta tat gca  1008
Glu Ala Leu Arg Leu Trp Pro Thr Ala Pro Ala Phe Ser Leu Tyr Ala
320             325             330             335

aaa gaa gat acg gtg ctt gga gga gaa tat cct tta gaa aaa ggc gac  1056
Lys Glu Asp Thr Val Leu Gly Gly Glu Tyr Pro Leu Glu Lys Gly Asp
                340             345             350

gaa cta atg gtt ctg att cct cag ctt cac cgt gat aaa aca att tgg  1104
Glu Leu Met Val Leu Ile Pro Gln Leu His Arg Asp Lys Thr Ile Trp
                355             360             365

gga gac gat gtg gaa gag ttc cgt cca gag cgt ttt gaa aat cca agt  1152
Gly Asp Asp Val Glu Glu Phe Arg Pro Glu Arg Phe Glu Asn Pro Ser
                370             375             380

gcg att ccg cag cat gcg ttt aaa ccg ttt gga aac ggt cag cgt gcg  1200
Ala Ile Pro Gln His Ala Phe Lys Pro Phe Gly Asn Gly Gln Arg Ala
                385             390             395

tgt atc ggt cag cag ttc gct ctt cat gaa gca acg ctg gta ctt ggt  1248
Cys Ile Gly Gln Gln Phe Ala Leu His Glu Ala Thr Leu Val Leu Gly
```

```
                400             405             410             415

atg atg cta aaa cac ttt gac ttt gaa gat cat aca aac tac gag ctg   1296
Met Met Leu Lys His Phe Asp Phe Glu Asp His Thr Asn Tyr Glu Leu

                420             425             430

gat att aaa gaa act tta acg tta aaa cct gaa ggc ttt gtg gta aaa   1344
Asp Ile Lys Glu Thr Leu Thr Leu Lys Pro Glu Gly Phe Val Val Lys

                435             440             445

gca aaa tcg aaa aaa att ccg ctt ggc ggt att cct tca cct agc act   1392
Ala Lys Ser Lys Lys Ile Pro Leu Gly Gly Ile Pro Ser Pro Ser Thr

                450             455             460

gaa cag tct gct aaa aaa gta cgc aaa aag gca gaa aac gct cat aat   1440
Glu Gln Ser Ala Lys Lys Val Arg Lys Lys Ala Glu Asn Ala His Asn

                465             470             475

acg ccg ctg ctt gtg cta tac ggt tca aat atg gga aca gct gaa gga   1488
Thr Pro Leu Leu Val Leu Tyr Gly Ser Asn Met Gly Thr Ala Glu Gly

                480             485             490             495

acg gcg cgt gat tta gca gat att gca atg agc aaa gga ttt gca ccg   1536
Thr Ala Arg Asp Leu Ala Asp Ile Ala Met Ser Lys Gly Phe Ala Pro

                500             505             510

cag gtc gca acg ctt gat tca cac gcc gga aat ctt ccg cgc gaa gga   1584
Gln Val Ala Thr Leu Asp Ser His Ala Gly Asn Leu Pro Arg Glu Gly

                515             520             525

gct gta tta att gta acg gcg tct tat aac ggt cat ccg cct gat aac   1632
Ala Val Leu Ile Val Thr Ala Ser Tyr Asn Gly His Pro Pro Asp Asn

                530             535             540

gca aag caa ttt gtc gac tgg tta gac caa gcg tct gct gat gaa gta   1680
Ala Lys Gln Phe Val Asp Trp Leu Asp Gln Ala Ser Ala Asp Glu Val

                545             550             555

aaa ggc gtt cgc tac tcc gta ttt gga tgc ggc gat aaa aac tgg gct   1728
Lys Gly Val Arg Tyr Ser Val Phe Gly Cys Gly Asp Lys Asn Trp Ala

                560             565             570             575
```

19

```
act acg tat caa aaa gtg cct gct ttt atc gat gaa acg ctt gcc gct   1776
Thr Thr Tyr Gln Lys Val Pro Ala Phe Ile Asp Glu Thr Leu Ala Ala
            580             585             590

aaa ggg gca gaa aac atc gct gac cgc ggt gaa gca gat gca agc gac   1824
Lys Gly Ala Glu Asn Ile Ala Asp Arg Gly Glu Ala Asp Ala Ser Asp
            595             600             605

gac ttt gaa ggc aca tat gaa gaa tgg cgt gaa cat atg tgg agt gac   1872
Asp Phe Glu Gly Thr Tyr Glu Glu Trp Arg Glu His Met Trp Ser Asp
            610             615             620

gta gca gcc tac ttt aac ctc gac att gaa aac agt gaa gat aat aaa   1920
Val Ala Ala Tyr Phe Asn Leu Asp Ile Glu Asn Ser Glu Asp Asn Lys
            625             630             635

tct act ctt tca ctt caa ttt gtc gac agc gcc gcg gat atg ccg ctt   1968
Ser Thr Leu Ser Leu Gln Phe Val Asp Ser Ala Ala Asp Met Pro Leu
640             645             650             655

gcg aaa atg cac ggt gcg ttt tca acg aac gtc gta gca agc aaa gaa   2016
Ala Lys Met His Gly Ala Phe Ser Thr Asn Val Val Ala Ser Lys Glu
            660             665             670

ctt caa cag cca ggc agt gca cga agc acg cga cat ctt gaa att gaa   2064
Leu Gln Gln Pro Gly Ser Ala Arg Ser Thr Arg His Leu Glu Ile Glu
            675             680             685

ctt cca aaa gaa gct tct tat caa gaa gga gat cat tta ggt gtt att   2112
Leu Pro Lys Glu Ala Ser Tyr Gln Glu Gly Asp His Leu Gly Val Ile
            690             695             700

cct cgc aac tat gaa gga ata gta aac cgt gta aca gca agg ttc ggc   2160
Pro Arg Asn Tyr Glu Gly Ile Val Asn Arg Val Thr Ala Arg Phe Gly
            705             710             715

cta gat gca tca cag caa atc cgt ctg gaa gca gaa gaa gaa aaa tta   2208
Leu Asp Ala Ser Gln Gln Ile Arg Leu Glu Ala Glu Glu Glu Lys Leu
720             725             730             735

gct cat ttg cca ctc gct aaa aca gta tcc gta gaa gag ctt ctg caa   2256
```

```
          Ala His Leu Pro Leu Ala Lys Thr Val Ser Val Glu Glu Leu Leu Gln
                      740                 745                 750

tac gtg gag ctt caa gat cct gtt acg cgc acg cag ctt cgc gca atg      2304
Tyr Val Glu Leu Gln Asp Pro Val Thr Arg Thr Gln Leu Arg Ala Met
            755                 760                 765

gct gct aaa acg gtc tgc ccg ccg cat aaa gta gag ctt gaa gcc ttg      2352
Ala Ala Lys Thr Val Cys Pro Pro His Lys Val Glu Leu Glu Ala Leu
            770                 775                 780

ctt gaa aag caa gcc tac aaa gaa caa gtg ctg gca aaa cgt tta aca      2400
Leu Glu Lys Gln Ala Tyr Lys Glu Gln Val Leu Ala Lys Arg Leu Thr
            785                 790                 795

atg ctt gaa ctg ctt gaa aaa tac ccg gcg tgt gaa atg aaa ttc agc      2448
Met Leu Glu Leu Leu Glu Lys Tyr Pro Ala Cys Glu Met Lys Phe Ser
800                 805                 810                 815

gaa ttt atc gcc ctt ctg cca agc ata cgc ccg cgc tat tac tcg att      2496
Glu Phe Ile Ala Leu Leu Pro Ser Ile Arg Pro Arg Tyr Tyr Ser Ile
                      820                 825                 830

tct tca tca cct cgt gtc gat gaa aaa caa gca agc atc acg gtc agc      2544
Ser Ser Ser Pro Arg Val Asp Glu Lys Gln Ala Ser Ile Thr Val Ser
                      835                 840                 845

gtt gtc tca gga gaa gcg tgg agc gga tat gga gaa tat aaa gga att      2592
Val Val Ser Gly Glu Ala Trp Ser Gly Tyr Gly Glu Tyr Lys Gly Ile
            850                 855                 860

gcg tcg aac tat ctt gcc gag ctg caa gaa gga gat acg att acg tgc      2640
Ala Ser Asn Tyr Leu Ala Glu Leu Gln Glu Gly Asp Thr Ile Thr Cys
            865                 870                 875

ttt att tcc aca ccg cag tca gaa ttt acg ctg cca aaa gac cct gaa      2688
Phe Ile Ser Thr Pro Gln Ser Glu Phe Thr Leu Pro Lys Asp Pro Glu
880                 885                 890                 895

acg ccg ctt atc atg gtc gga ccg gga aca ggc gtc gcg ccg ttt aga      2736
Thr Pro Leu Ile Met Val Gly Pro Gly Thr Gly Val Ala Pro Phe Arg
```

```
                    900                 905                 910
ggc ttt gtg cag gcg cgc aaa cag cta aaa gaa caa gga cag tca ctt    2784
Gly Phe Val Gln Ala Arg Lys Gln Leu Lys Glu Gln Gly Gln Ser Leu

                    915                 920                 925
gga gaa gca cat tta tac ttc ggc tgc cgt tca cct cat gaa gac tat    2832
Gly Glu Ala His Leu Tyr Phe Gly Cys Arg Ser Pro His Glu Asp Tyr

                    930                 935                 940
ctg tat caa gaa gag ctt gaa aac gcc caa agc gaa ggc atc att acg    2880
Leu Tyr Gln Glu Glu Leu Glu Asn Ala Gln Ser Glu Gly Ile Ile Thr

                    945                 950                 955
ctt cat acc gct ttt tct cgc atg cca aat cag ccg aaa aca tac gtt    2928
Leu His Thr Ala Phe Ser Arg Met Pro Asn Gln Pro Lys Thr Tyr Val

960                 965                 970                 975
cag cac gta atg gaa caa gac ggc aag aaa ttg att gaa ctt ctt gat    2976
Gln His Val Met Glu Gln Asp Gly Lys Lys Leu Ile Glu Leu Leu Asp

                    980                 985                 990
caa gga gcg cac ttc tat att tgc gga gac gga agc caa atg gca cct    3024
Gln Gly Ala His Phe Tyr Ile Cys Gly Asp Gly Ser Gln Met Ala Pro

                    995                 1000                1005
gcc gtt gaa gca acg ctt atg aaa agc tat gct gac gtt cac caa gtg    3072
Ala Val Glu Ala Thr Leu Met Lys Ser Tyr Ala Asp Val His Gln Val

                    1010                1015                1020
agt gaa gca gac gct cgc tta tgg ctg cag cag cta gaa gaa aaa ggc    3120
Ser Glu Ala Asp Ala Arg Leu Trp Leu Gln Gln Leu Glu Glu Lys Gly

                    1025                1030                1035
cga tac gca aaa gac gtg tgg gct ggg taa                            3150
Arg Tyr Ala Lys Asp Val Trp Ala Gly

                    1040                1045
```

<210> 2
<211> 1048
<212> PRT
<213> Bacillus megaterium
<400> 2

Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
                20                  25                  30

Ala Asp Glu Leu Gly Glu Ile Phe Lys Phe Glu Ala Pro Gly Arg Val
                35                  40                  45

Thr Arg Tyr Leu Ser Ser Gln Arg Leu Ile Lys Glu Ala Cys Asp Glu
                50                  55                  60

Ser Arg Phe Asp Lys Asn Leu Ser Gln Ala Leu Lys Phe Val Arg Asp
65                  70                  75                  80

Phe Ala Gly Asp Gly Leu Phe Thr Ser Trp Thr His Glu Lys Asn Trp
                85                  90                  95

Lys Lys Ala His Asn Ile Leu Leu Pro Ser Phe Ser Gln Gln Ala Met
                100                 105                 110

Lys Gly Tyr His Ala Met Met Val Asp Ile Ala Val Gln Leu Val Gln
                115                 120                 125

Lys Trp Glu Arg Leu Asn Ala Asp Glu His Ile Glu Val Pro Glu Asp
                130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
                180                 185                 190

Pro Asp Asp Pro Ala Tyr Asp Glu Asn Lys Arg Gln Phe Gln Glu Asp
                195                 200                 205

Ile Lys Val Met Asn Asp Leu Val Asp Lys Ile Ile Ala Asp Arg Lys
                210                 215                 220

Ala Ser Gly Glu Gln Ser Asp Asp Leu Leu Thr His Met Leu Asn Gly

                225                 230                 235                 240

Lys Asp Pro Glu Thr Gly Glu Pro Leu Asp Asp Glu Asn Ile Arg Tyr
                        245                 250                 255

Gln Ile Ile Thr Phe Leu Ile Ala Gly His Glu Thr Thr Ser Gly Leu
                    260                 265                 270

Leu Ser Phe Ala Leu Tyr Phe Leu Val Lys Asn Pro His Val Leu Gln
                275                 280                 285

Lys Ala Ala Glu Glu Ala Ala Arg Val Leu Val Asp Pro Val Pro Ser
                290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                        325                 330                 335

Glu Asp Thr Val Leu Gly Gly Glu Tyr Pro Leu Glu Lys Gly Asp Glu
                    340                 345                 350

Leu Met Val Leu Ile Pro Gln Leu His Arg Asp Lys Thr Ile Trp Gly
                355                 360                 365

Asp Asp Val Glu Glu Phe Arg Pro Glu Arg Phe Glu Asn Pro Ser Ala
                370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                        405                 410                 415

Met Leu Lys His Phe Asp Phe Glu Asp His Thr Asn Tyr Glu Leu Asp
                420                 425                 430

Ile Lys Glu Thr Leu Thr Leu Lys Pro Glu Gly Phe Val Val Lys Ala
                435                 440                 445

Lys Ser Lys Lys Ile Pro Leu Gly Gly Ile Pro Ser Pro Ser Thr Glu
                450                 455                 460

Gln Ser Ala Lys Lys Val Arg Lys Lys Ala Glu Asn Ala His Asn Thr
465                 470                 475                 480

```
Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
                500                 505                 510

Val Ala Thr Leu Asp Ser His Ala Gly Asn Leu Pro Arg Glu Gly Ala
            515                 520                 525

Val Leu Ile Val Thr Ala Ser Tyr Asn Gly His Pro Pro Asp Asn Ala
        530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
                580                 585                 590

Gly Ala Glu Asn Ile Ala Asp Arg Gly Glu Ala Asp Ala Ser Asp Asp
            595                 600                 605

Phe Glu Gly Thr Tyr Glu Glu Trp Arg Glu His Met Trp Ser Asp Val
            610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
                660                 665                 670

Gln Gln Pro Gly Ser Ala Arg Ser Thr Arg His Leu Glu Ile Glu Leu
            675                 680                 685

Pro Lys Glu Ala Ser Tyr Gln Glu Gly Asp His Leu Gly Val Ile Pro
            690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
```

725 730 735

His Leu Pro Leu Ala Lys Thr Val Ser Val Glu Glu Leu Leu Gln Tyr

740 745 750

Val Glu Leu Gln Asp Pro Val Thr Arg Thr Gln Leu Arg Ala Met Ala

755 760 765

Ala Lys Thr Val Cys Pro Pro His Lys Val Glu Leu Glu Ala Leu Leu

770 775 780

Glu Lys Gln Ala Tyr Lys Glu Gln Val Leu Ala Lys Arg Leu Thr Met

785 790 795 800

Leu Glu Leu Leu Glu Lys Tyr Pro Ala Cys Glu Met Lys Phe Ser Glu

805 810 815

Phe Ile Ala Leu Leu Pro Ser Ile Arg Pro Arg Tyr Tyr Ser Ile Ser

820 825 830

Ser Ser Pro Arg Val Asp Glu Lys Gln Ala Ser Ile Thr Val Ser Val

835 840 845

Val Ser Gly Glu Ala Trp Ser Gly Tyr Gly Glu Tyr Lys Gly Ile Ala

850 855 860

Ser Asn Tyr Leu Ala Glu Leu Gln Glu Gly Asp Thr Ile Thr Cys Phe

865 870 875 880

Ile Ser Thr Pro Gln Ser Glu Phe Thr Leu Pro Lys Asp Pro Glu Thr

885 890 895

Pro Leu Ile Met Val Gly Pro Gly Thr Gly Val Ala Pro Phe Arg Gly

900 905 910

Phe Val Gln Ala Arg Lys Gln Leu Lys Glu Gln Gly Gln Ser Leu Gly

915 920 925

Glu Ala His Leu Tyr Phe Gly Cys Arg Ser Pro His Glu Asp Tyr Leu

930 935 940

Tyr Gln Glu Glu Leu Glu Asn Ala Gln Ser Glu Gly Ile Ile Thr Leu

945 950 955 960

His Thr Ala Phe Ser Arg Met Pro Asn Gln Pro Lys Thr Tyr Val Gln

965 970 975

27

His Val Met Glu Gln Asp Gly Lys Lys Leu Ile Glu Leu Leu Asp Gln

980 985 990

Gly Ala His Phe Tyr Ile Cys Gly Asp Gly Ser Gln Met Ala Pro Ala

995 1000 1005

Val Glu Ala Thr Leu Met Lys Ser Tyr Ala Asp Val His Gln Val Ser

1010 1015 1020

Glu Ala Asp Ala Arg Leu Trp Leu Gln Gln Leu Glu Glu Lys Gly Arg

025 1030 1035 1040

Tyr Ala Lys Asp Val Trp Ala Gly

1045

<210> 3
<211> 30
<212> DNA
<213> Künstliche Sequenz
<220>
<223> Beschreibung der künstlichen Sequenz: PCR-Primer
<400> 3
gcaggagacg ggttgnnnac aagctggacg          30
<210> 4
<211> 30
<212> DNA
<213> Künstliche Sequenz
<220>
<223> Beschreibung der künstlichen Sequenz: PCR-Primer
<400> 4
cgtccagctt gtnnncaacc cgtctcctgc          30
<210> 5
<211> 34
<212> DNA
<213> Künstliche Sequenz
<220>
<223> Beschreibung der künstlichen Sequenz: PCR-Primer
<400> 5
gaagcaatga acaagnnnca gcgagcaaat ccag          34
<210> 6
<211> 34
<212> DNA
<213> Künstliche Sequenz
<220>
<223> Beschreibung der künstlichen Sequenz: PCR-Primer
<400> 6
ctggatttgc tcgctgnnnc ttgttcattg cttc          34
<210> 7
<211> 41
<212> DNA
<213> Künstliche Sequenz
<220>

<223> Beschreibung der künstlichen Sequenz: PCR-Primer

<400> 7

gctttgataa aaacttaaag tcaannnctt aaatttgtac g   41

<210> 8

<211> 40

<212> DNA

<213> Künstliche Sequenz

<220>

<223> Beschreibung der künstlichen Sequenz: PCR-Primer

<400> 8

cgtacaaatt taagnnnttg acttaagttt ttatcaaagc   40

<210> 9

<211> 37

<212> DNA

<213> Künstliche Sequenz

<220>

<223> Beschreibung der künstlichen Sequenz: PCR-Primer

<400> 9

gttattaaac acagataaan nngttcaagc tttgatg   37

<210> 10

<211> 37

<212> DNA

<213> Künstliche Sequenz

<220>

<223> Beschreibung der künstlichen Sequenz: PCR-Primer

<400> 10

catcaaagct tgaacnnntt tatctgtgtt taataac   37

<210> 11

<211> 37

<212> DNA

<213> Künstliche Sequenz

<220>

<223> Beschreibung der künstlichen Sequenz: PCR-Primer

<400> 11

gttattaaac acagataaac cgnnncaagc tttgatg   37

<210> 12

<211> 37

<212> DNA

<213> Künstliche Sequenz

<220>

<223> Beschreibung der künstlichen Sequenz: PCR-Primer

<400> 12

catcaaagct tgnnncggtt tatctgtgtt taataac   37

<210> 13

<211> 33

<212> DNA

<213> Künstliche Sequenz

<220>

<223> Beschreibung der künstlichen Sequenz: PCR-Primer

<400> 13

cgaggcgcct ggtnnngtaa cgcgctactt atc   33

<210> 14

<211> 33

<212> DNA

<213> Künstliche Sequenz

<220>

<223> Beschreibung der künstlichen Sequenz: PCR-Primer

<400> 14

gataagtagc gcgttacnnn accaggcgcc tcg          33

<210> 15

<211> 34

<212> DNA

<213> Künstliche Sequenz

<220>

<223> Beschreibung der künstlichen Sequenz: PCR-Primer

<400> 15

cctggtcgtg taacgcgcnn nttatcaagt cagc          34

<210> 16

<211> 34

<212> DNA

<213> Künstliche Sequenz

<220>

<223> Beschreibung der künstlichen Sequenz: PCR-Primer

<400> 16

gctgacttga taannngcgc gttacacgac cagg          34

<210> 17

<211> 40

<212> DNA

<213> Künstliche Sequenz

<220>

<223> Beschreibung der künstlichen Sequenz: PCR-Primer

<400> 17

gctttgataa aaacttannn caagcgctta aatttgtacg          40

<210> 18

<211> 40

<212> DNA

<213> Künstliche Sequenz

**<220>**

<223> Beschreibung der künstlichen Sequenz: PCR-Primer

<400> 18

cgtacaaatt taagcgcttg nnntaagttt ttatcaaagc          40

<210> 19

<211> 30

<212> DNA

<213> Künstliche Sequenz

<220>

<223> Beschreibung der künstlichen Sequenz: PCR-Primer

<400> 19

ggcgacgaac tannngttct gattcctcag          30

<210> 20

<211> 30

<212> DNA

<213> Künstliche Sequenz

<220>

<223> Beschreibung der künstlichen Sequenz: PCR-Primer

<400> 20

ctgaggaatc agaacnnnta gttcgtcgcc          30

**Beschreibung**

Die vorliegende Erfindung betrifft modifizierte Cytochrom P450-Monooxygenasen mit verändertem Substratprofil, dafür kodierende Nukleinsäuresequenzen, Expressionskonstrukte und Vektoren, rekombinante Mikroorganismen, welche diese Vektoren enthalten sowie Verfahren zur mikrobiologischen Herstellung terminal oder subterminal hydroxylierter aliphatischer Carbonsäuren.

Die Monooxygenase mit der Bezeichnung P450 BM-3 ist ein Cytochrom P450-Enzym aus *Bacillus megaterium* und besitzt eine ausgeprägte Sequenzhomologie mit P450-Enzymen aus Säugern (1). Aufgrund dieser Übereinstimmungen stellt P450 BM-3 ein ausgezeichnetes Modellsystem für diese Klasse von P450-Enzymen dar. P450 BM-3 hydroxyliert in erster Linie langkettige gesättigte Fettsäuren an ihrem $\omega$-1-, $\omega$-2- und $\omega$-3-Kohlenstoffatom. Amide oder Alkoholanaloga und Epoxide langkettiger ungesättigter Fettsäuren werden ebenfalls umgesetzt (1-3). Die katalytische Aktivität für gesättigte Fettsäuren ist abhängig von der Kettenlänge, wobei das Kettenlängen-Optimum bei 14 bis 16 Kohlenstoffatomen liegt. Das Enzym zeigt keine katalytische Aktivität für Fettsäuren mit einer Kettenlänge von weniger als 12 Kohlenstoffatomen (1).

Zusammenfassung der Erfindung

Aufgabe der vorliegenden Erfindung war es daher, Cytochrom P450-Monooxygenase-Mutanten bereitzustellen, welche im Vergleich zum Wildtyp-Enzym ein modifiziertes Substratprofil zeigen. Insbesondere sollten neue Mutanten bereitgestellt werden, welche gesättigte aliphatische Carbonsäuren in anderer Kettenposition hydroxylieren und/oder eine veränderte Substratspezifität besitzen. Vor allem sollten Mutanten bereitgestellt werden, welche katalytische Aktivität gegenüber aliphatischen Carbonsäuren mittlerer Kettenlänge, insbesondere mit einer Kettenlänge von 8 bis 12, wie z. B. 8 bis 10 Kohlenstoffatomen besitzen, und diese subterminal, vor allem an den Positionen $\omega$-1, $\omega$-2 und/oder $\omega$-3 hydroxylieren.

Diese Aufgaben wurden überraschenderweise gelöst durch Bereitstellung modifizierter Cytochrom P450-Monooxygenasen, welche durch eine Kombination aus gerichteter Evolution und ortsspezifischer Mutagenese ihres Substrat-bindenden Bereichs im Vergleich zum Wildtyp ein verändertes Reaktivitätsmuster bzw. Substratprofil bei der terminalen und/oder subterminalen enzymatischen Hydroxylierung von aliphatischen Carbonsäuren zeigen.

Detaillierte Beschreibung der Erfindung

Für erfindungsgemäße Mutanten ist im Vergleich zum Wildtyp-Enzym ein "verändertes Substratprofil" zu beobachten. Ein "verändertes Substratprofil" bedeutet in Rahmen der vorliegenden Erfindung a) eine Verbesserung der Reaktivität, wie z. B. eine Erhöhung der spezifischen Aktivität (ausgedrückt als nmol umgesetzte Carbonsäure/Minute/nmol P450-Enzym) und/oder wenigstens eines kinetischen Parameters, ausgewählt unter Kcat, Km und Kcat/Km, z.B. um mindestens 1 %, wie z. B. 10 bis 1000 %, 10 bis 500 %, oder 10 bis 100 %, der Mutante gegenüber zumindest einer hydroxylierbaren aliphatischen Carbonsäure oder einem hydroxylierbaren Derivat einer aliphatischen Carbonsäure und/oder b) eine Veränderung, insbesondere Erhöhung, der Regioselektivität bei der Carbonsäure-Hydroxylierung. So ist z.B. eine Verschiebung der bevorzugten terminalen oder subterminalen ($\omega$-1, $\omega$-2, $\omega$-3, $\omega$-4, insbesondere $\omega$-1 bis $\omega$-3) Hydroxylierungsposition an wenigstens einer hydroxylierbaren Carbonsäure oder einem hydroxylierbaren Derivat einer aliphatischen Carbonsäure. Hydroxylierbare aliphatische Carbonsäuren oder Derivate davon, bei denen erfindungsgemäß ein "verändertes Substratprofil" zu beobachten ist, sind verzweigte oder vorzugsweise geradkettige Carbonsäuren mit 8 bis 30 Kohlenstoffatomen. Die erfindungsgemäße Veränderung des Substratprofils kann dabei über den gesamten Größenbereich (d. h. $C_8$-$C_{30}$) oder nur in Teilbereichen, z. B. bei $C_8$-$C_{12}$-, -$C_{10}$-$C_{12}$-, $C_{12}$-$C_{30}$-, $C_{12}$-$C_{25}$- oder $C_{12}$-$C_{20}$-Carbonsäuren oder bei einzelnen Carbonsäuren aus diesen Teilbereichen ausgeprägt sein.

Als nichtlimitierende Beispiele für erfindungsgemäß hydroxylierbare Carbonsäuren sind zu nennen: Capryl-, Pelargon-, Caprin-, Undecan-, Laurin-, Tridecan-, Myristin-, Pentadecan-, Palmitin-, Margarin-, Stearin-, Nonadecan-, Arachin-, Behen-, Lignocerin-, Cerotin- und Melissinsäure. Beispiele für geeignete Carbonsäurederivate sind $C_1$-$C_4$-Alkylester, Amide oder Anhydride mit vorzugsweise kurzkettigen $C_1$-$C_4$-Carbonsäuren.

Die erfindungsgemäßen Monooxygenasen sind vorzugsweise abgeleitet von Cytochrom P450 Monooxygenasen der Enzymklasse E.C. 1.14.-.-, insbesondere aus der P450-Familie CYP102, und sind eukaryotischen oder prokaryotischen, insbesondere bakteriellen Ursprungs.

Eine besonders bevorzugte Gruppe von Mutanten ist abgeleitet von Cytochrom P450 Monooxygenase BM-3 aus *Bacillus megaterium* mit einer Aminosäuresequenz gemäß SEQ ID NO:2, welche wenigstens eine funktionale Mutation in einem der folgenden Aminosäuresequenzbereiche aufweist: 24-28, 45-51, 70-72, 73-82 (helix 5), 86-88 (helix 6), 172-224 (F/G-loop) und 352-356 ($\beta$-strand 8), mit der Maßgabe, dass mehr als einer dieser Bereiche mutiert ist, wenn das Enzym die Mutation F87A trägt; sowie funktionale Äquivalente dieser Mutanten.

Die jeweilige Mutation wird im Rahmen der vorliegenden Beschreibung im Aminosäure-Einbuchstabencode angegeben. Vor der Zahl, welche die Sequenzposition der Mutation bezeichnet, ist die ursprüngliche Aminosäure, nach der Zahl die modifizierte Aminosäure angegeben.

Eine "funktionale Mutation" im Sinne der vorliegenden Erfindung umfasst einen Aminosäureaustausch in den genannten Sequenzbereichen, welcher zu einem "veränderten Reaktivitätsmuster" bzw. "veränderten Substratprofil" gemäß obiger Definition führt.

Gegenstand der Erfindung sind modifizierte Cytochrom P450 Monooxygenasen gemäß Anspruch 1.

Bevorzugte Mutanten weist wenigstens eines der folgenden Aminosäuresubstitutionsmuster auf:

    a) F87A, L188K;
    b) F87V, L188K;
    c) F87A, L188K; A74G;
    d) F87V, L188K, A74G;
    e) F87A, L188K, A74G, R47F;
    f) F87V, L188K, A74G, R47F;
    g) F87A, L188K, A74G, R47F, V26T; oder
    h) F87V, L188K, A74G, R47F, V26T;

Gegenstand der Erfindung sind außerdem Nukleinsäuresequenzen, kodierend für eine mutierte Monooxygenase oder ein "funktionales Äquivalent" gemäß obiger Definition. Diese Sequenzen sind vorzugsweise von SEQ ID NO: 1 durch Austausch von Codons entsprechend der obigen Aminosäuresubstitutionsmuster erhältlich.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten davon. Erfindungsgemäß mit umfasst sind außerdem durch Degeneration des genetischen Codes (d. h. ohne Veränderung der korrespondierenden Aminosäuresequenz) oder konservative Nukleotidsubstitution (d. h. die korrespondierende Aminosäure wird durch eine andere Aminosäure gleicher Ladung, Größe, Polarität und/oder Löslichkeit ersetzt) erhaltene Abwandlungen der Nukleinsäuresequenzen, ebenso wie durch Nukleotidaddition, -insertion, -inversion oder -deletion veränderte Sequenzen, welche für eine erfindungsgemäße Monooxygenase mit "verändertem Substratprofil" kodieren, sowie die korrespondierenden komplemetären Sequenzen.

Gegenstand der Erfindung sind außerdem Expressionskonstrukte, enthaltend unter der genetischen Kontrolle regulativer Nukleinsäuresequenzen eine für eine erfindungsgemäße Mutante kodierende Nukleinsäuresequenz; sowie Vektoren, umfassend wenigstens eines dieser Expressionskonstrukte.

Vorzugsweise umfassen die erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer operativen Verknüpfung versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Translationsverstärker, Enhancer, Polyadenylierungssignale, Selektionsmarker, Amplifikationssignale, Replikationsursprünge und dergleichen.

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für Promotoren sind: cos-, tac-, trp-, tet-, trp-tet-, lpp-, lac-, lpp-lac-, lacIq-, T7-, T5-, T3-, gal-, trc-, ara-, SP6-, 1-PR- oder 1-PL-Promotor, die vorteilhafterweise in gramnegativen Bakterien Anwendung finden; sowie die gram-positiven Promotoren amy und SP02, die Hefepromotoren ADC1, MFa , AC, P-60, CYC1, GAPDH oder die Pflanzenpromotoren CaMV/35S, SSU, OCS, lib4, usp, STLS1, B33, nos oder der Ubiquitin- oder Phaseolin-Promotor.

Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors mit einer geeigneten Monooxygenase-Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Mit Hilfe der erfindungsgemäßen Vektoren sind rekombinante Mikroorganismen herstellbar, welche beispielsweise mit wenigstens einem erfindungsgemäßen Vektor transformierbar sind und zur Produktion der Mutanten eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden verwendet, wie beispielsweise Co-Präzipitation, Protoplastenfusion, Elektroporation, retrovirale Transfektion und dergleichen, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, beschrieben.

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen Escherichia, wie z. B. Escherichia coli, Streptomyces, Bacillus oder Pseudomonas, eukaryotische Mikroorganismen, wie Saccharomyces cerevisiae, Aspergillus, höhere eukaryotische Zellen aus Tieren oder Pflanzen, beispielsweise Sf9 oder CHO-Zellen.

Gewünschtenfalls kann das Genprodukt auch in transgenen Organismen wie transgenen Tieren, wie insbesondere Mäusen, Schafen oder transgenen Pflanzen zur Expression gebracht werden. Bei den transgenen Organismen kann es sich auch um sogenannte Knock-Out Tiere oder Pflanzen handeln, in denen das korrespondierende endo-gene Gen ausgeschaltet wurde, wie z. B. durch Mutation oder partielle oder vollständige Deletion.

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer zellsortierung selektiert werden. Erfolgreich mit einem Vektor transformierte Mikroorganismen, die ein entsprechendes Antibiotikaresistenzgen (z. B. G418 oder Hygromycin) tragen, lassen sich durch entsprechende Antibiotika-enthaltende Medien oder Nährböden selektieren. Markerproteine, die an der Zelloberfläche präsentiert werden, können zur Selektion mittels Affinitätschromatographie genutzt werden.

Die Kombination aus den Wirtsorganismen und den zu den Organismen passenden Vektoren, wie Plasmide, Viren oder Phagen, wie beispielsweise Plasmide mit dem RNA-Polymerase/Promoter-System, die Phagen $\lambda$, $\mu$ oder andere temperente Phagen oder Transposons und/oder weiteren vorteilhaften regulatorischen Sequenzen bildet ein Expressionssystem. Beispielsweise ist unter dem Begriff "Expressionssystem" die Kombination aus Säugetierzellen, wie CHO-Zellen, und Vektoren, wie pcDNA3neo-Vektor, die für Säugetierzellen geeignet sind, zu verstehen.

Wie oben beschrieben, kann das Genprodukt vorteilhaft auch in trans genen Tieren, z. B. Mäusen, Schafen oder transgenen Pflanzen zur Expression gebracht werden. Ebenso ist es möglich, zellfreie Translationssysteme mit der von der Nukleinsäure abgeleiteten RNA zu programmieren.

Gegenstand der Erfindung sind weiterhin Verfahren zur Herstellung einer erfindungsgemäßen Monooxygenase, wobei man einen Monooxygenase-produzierenden Mikroorganismus kultiviert, gegebenenfalls die Expression der Monooxygenase induziert und die Monooxygenase aus der Kultur isoliert. Die erfindungsgemäße Monooxygenase kann so auch in großtechnischem Maßstab produziert werden, falls dies erwünscht ist.

Der Mikroorganismus kann nach bekannten Verfahren kultiviert und fermentiert werden. Bakterien können beispielsweise in TB- oder LB-Medium und bei einer Temperatur von 20 bis 40 °C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Monooxygenase nicht in das Kulturmedium sezerniert wird, aufgeschlossen und die Monooxygenase nach bekannten Proteinisolierungsverfahren aus dem Lysat gewonnen. Die Zellen können wahlweise durch hochfrequenten Ultraschall, durch hohen Druck, wie z. B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Monooxygenase kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Besonders vorteilhaft ist es, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die einer einfacheren Reinigung dienen. Derartige geeignete Modifikationen sind beispielsweise als Anker fungierende sogenannte "Tags", wie z. B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z. B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Gegenstand der Erfindung sind außerdem biochemische Verfahren zur enzymatischen Herstellung von terminal oder subterminal ($\omega$-1 bis w-4) hydroxylierten aliphatischen Carbonsäuren, die dadurch gekennzeichnet sind, dass man

a1) einen erfindungsgemäßen rekombinanten Mikroorganismus in Gegenwart eines Kulturmediums, das wenigstens eine hydroxylierbare Carbonsäure oder wenigstens ein hydroxylierbares Carbonsäurederivat enthält, aerob kultiviert; oder

a2) ein Reaktionsmedium, enthaltend wenigstens eine hydroxylierbare Carbonsäure oder wenigstens ein hydroxylierbares Carbonsäurederivat, mit einer erfindungsgemäßen Mutante aerob inkubiert; und

b) das gebildete hydroxylierte Produkt oder Produktgemisch aus dem Medium isoliert.

Im erfindungsgemäßen Verfahren sind die Carbonsäuren per se, oder Derivate davon, wie insbesondere $C_1$-$C_4$-Alkylester oder Carbonsäureamide, einsetzbar.

Bevorzugt werden im erfindungsgemäßen Verfahren als hydroxylierbare Carbonsäure $C_8$-$C_{30}$-Monocarbonsäuren oder Derivate davon einsetzt.

Besonders bevorzugt verwendet man im erfindungsgemäßen Verfahren als hydroxylierbare Carbonsäure eine $C_8$-$C_{12}$-Monocarbonsäure oder ein Derivat davon und als Monooxygenase eine Mutante gemäß Anspruch 1.

Die erfindungsgemäße Oxidationsreaktion wird gewöhnlich in Gegenwart von Luftsauerstoff sowie in Gegenwart eines Elektronendonors (oder Reduktionsäquivalents), wie insbesondere NADH, NADPH und Zn/Co(III)sepulchrat, durchgeführt. Die Verwendung von Zn/Co(III)sepulchrat ist beispielsweise in der DE-A-199 35 115 beschrieben, worauf hiermit ausdrücklich Bezug genommen wird.

Wird die erfindungsgemäße Hydroxylierung mit einem rekombinanten Mikroorganismus durchgeführt, so erfolgt vorzugsweise zunächst die Kultivierung der Mikroorganismen in Gegenwart von Sauerstoff und in einem Komplexmedium, wie z.B. TB- oder LB- Medium bei einer Kultivierungstemperatur von etwa 20 bis 40 °C und einem pH-Wert von etwa 6 bis 9, bis eine ausreichende Zelldichte erreicht ist. Die Zugabe von exogenem Substrat erfolgt je nach Bedarf. Um die Oxidationsreaktion besser steuern zu können, bevorzugt man die Verwendung eines induzierbaren, insbesondere temperaturinduzierbaren, Promotors. Man erhöht dabei die Temperatur auf die erforderliche Induktionstemperatur, z.B. 42 °C beim $P_rP_1$-Promotor, behält dies über einen ausreichenden Zeitraum, z. B. 1 bis 10 oder 5 bis 6 Stunden, zur Expression der Monooxygenase-Aktivität bei und verringert anschließend der Temperatur wieder einer Wert von etwa 30 bis 40°C. Die Kultivierung wird dann in Gegenwart von Sauerstoff 12 Stunden bis 3 Tage fortgesetzt.

Wird die erfindungsgemäße Oxidation dagegen mit gereinigtem oder angereichertem Enzym durchgeführt so löst man die erfindungsgemäße Enzymmutante in einem exogenes Substrat enthaltenden Medium (etwa 0,01 bis 10 mM, oder 0,05 bis 5 mM), und führt die Umsetzung, vorzugsweise in Gegenwart von Sauerstoff, bei einer Temperatur von etwa 10 bis 50 °C, wie z.B. 30 bis 40 °C, und einem pH-Wert von etwa 6 bis 9 (wie z.B. eingestellt mit 100 bis 200 mM Phosphat- oder Tris-Puffer), sowie in Gegenwart eines Reduktionsmittels durch, wobei das Substrat-haltige Medium außerdem bezogen auf das zu oxidierende Substrat einen etwa 1-bis 100-fachen molaren Überschuß an Reduktionsäquivalenten qufweisen kann. Die Zugabe des Reduktionsmittels kann falls erforderlich portionsweise erfolgen.

Falls erforderlich kann Sauerstoff in an sich bekannter Weise in das Reaktionsmedium eingeleitet werden.

Werden die erfindungsgemäßen Verfahren mit angereichertem oder gereinigtem Enzym durchgeführt, so kann man beispielsweise folgende Reaktionbedingungen einstellen:

| | |
|---|---|
| Substratkonzentration: | 0,1 bis 20 mg/ml |
| Enzymkonzentration: | 0,1 bis 10 mg/ml |
| Reaktionstemperatur: | 20 bis 40 °C |
| pH: | 6 bis 8 |
| Puffer: | 0,05 bis 0,2 M Kaliumphosphat, oder Tris/HCl |
| Elektronendonor: | wird bevorzugt portionsweise zugegeben (Anfangskonzentration etwa 0,1 bis 2 mg/ml). |

Vor dem Start der Reaktion durch Zugabe der Elektronendonors kann zur Aktivitätssteigerung Aceton in einer Konzentration von 1 bis 5 % (v/v) zugegeben und kurzzeitig (1 bis 5 Minuten) vorinkubiert werden (bei etwa 20 bis 40 °C).

Der Fachmann kann, abweichend davon, durch routinemäßige Versuche die jeweiligen Reaktionsbedingungen optimieren.

Zur Erzeugung von Varianten mit modifizierten enzymatischen Eigenschaften stehen im Rahmen des Protein-Engineerings zwei unterschiedliche Strategien zur Verfügung: zum einen die Methode des "rationalen Designs" (4) sowie die Methode der "gezielten Evolution" (5-7). Für ein erfolgreiches rationales Design ist ein hochaufgelöstes dreidimensionales Strukturmodell sowie eine vertiefte Kenntnis des Enzymmechanismus von zentraler Bedeutung. Während für das rationale Design die Fähigkeit gezeigt wurde, Enzymmutanten zu erzeugen, welche hohe Aktivität gegenüber nichtnatürlichen Substraten besitzen (4) ist der Effekt punktförmiger einzelner Aminosäuresubstitutionen auf die Stabilität, Aktivität und Spezifität der Mutanten häufig nicht vorhersagbar.

Obwohl Röntgenstrukturen von P450 BM-3 in der Protein-Data Bank (8) hinterlegt wurden, ist die Konformation von Substrat und P450 während des kritischen Hydroxylierungsschrittes weiterhin unklar (9-11). Für die Methode der gezielten Evolution ist eine effiziente Nachweismethode für ein schnelles Screening großer Bibliotheken von Zufallsmutanten von zentraler Bedeutung. Ein optischer Test für die P450 BM-3-Mutante F87A, welche $\omega$-para-Nitrophenoxycarbonsäuren (pNCA) verwendet, hat sich als brauchbar erwiesen (12, 13). Die Einzelmutante F87A verschiebt die Hydroxylierungsposition von Fettsäuren von der Position $\omega$-1, $\omega$-2 und $\omega$-3 in Richtung der $\omega$-Position (13) und führt außerdem zu einer vollständigen Umwandlung von 12-pNCA im Vergleich zu einer 33 %igen Umwandlung, welche man für das wildtyp-Enzym beobachtet (12). Da jedoch die Monooxygenase-Domäne von P450 BM-3 mehr als 400 Aminosäuren umfasst, kommt das Screening einer Bibliothek von Zufallsmutanten der Suche nach einer Nadel im Heuhaufen gleich.

Zur Verbesserung der Effizienz der Suchprozedur wurde erfindungsgemäß das Verfahren des rationalen Designs mit dem Verfahren der gezielten Evolution kombiniert, um Mutanten mit modifizierter Reaktivität, insbesondere zur Erzeugung von Mutanten mit Spezifität für Fettsäuren mittlerer Kettenlänge, zu erzeugen. Die erfindungsgemäße Methode der "rationalen Evolution" basiert auf einem Computer-unterstützten Protein-Modeling, um eine virtuelle Zufalls-Bibliothek zu erzeugen und um Reste zu identifizieren, die mit hoher Wahrscheinlichkeit die gewünschten Eigenschaften beeinflussen. Eine Unter-Bibliothek wird durch Randomisierung dieser Reste erzeugt und auf positive Mutanten gescreent. Ausgehend von einem Strukturmodell beginnt man mit der Bestimmung von Resten, welche möglicherweise für die Kettenlängenspezifität von Bedeutung sind. Zur Erzeugung von Mutanten mit verbesserten Eigenschaften wird die durch das Modell vorgeschlagene Mutationsstelle mit Hilfe der Sättigungsmutagene modifiziert. Anschließend werden dann die Einzelmutanten mit den besten Eigenschaften unter Berücksichtigung des rationalen Designs miteinander kombiniert. Unter Anwendung dieser kombinierten Strategie konnte insbesondere die Aktivität von P450 BM-3 für Substrate mittlerer Kettenlänge verbessert werden.

Unter Anwendung dieser Strategie und aufgrund der Sequenzhomologie zu P450-Enzymen anderen Ursprungs wird es dem Fachmann ermöglicht in analoger Weise weitere Mutanten herzustellen, welche ebenfalls Gegenstand der Erfindung sind.

Die vorliegende Erfindung wird nun durch folgende experimentelle Beschreibung und unter Bezugnahme auf beiliegende Figuren näher erläutert. Dabei zeigt:

Figur 1 die schrittweise Optimierung von P450 BM-3 für das neue Substrat 8-pNCA. Die katalytische Effizienz gegenüber 8-pNCA ist für jede Mutante (in der Einheit $s^{-1}M^{-1}$) angegeben; und

Figur 2 ein Modell des Komplexes der P450 BM-3-Mutante LARVF mit dem Substrat 8-pNCA. Die Seitenketten, welche die fünf besten Mutationspositionen aufweisen und die zur Mutante LARVF kombiniert wurden, sind dargestellt (V26T, R47F, A74G, F87V, L188K).

Methode 1: Modellierung des Substrat-P450 BM-3-Komplexes

Die Modellierung des Substrat-Enzym-Komplexes basierte auf der kristallographisch bestimmten Struktur von P450 BM-3, komplexiert mit Palmitoleinsäure (9). Diese Struktur ist bei der Protein-Data Bank hinterlegt (8). Die beschriebene Kristallstruktur mit einer Auflösung von 2,9 Å enthält vier Moleküle in einer asymmetrischen Einheitszelle. Kette A wurde als Referenz für den modellierten Komplex ausgewählt. Als Substratmolekül wurde ein Modell von 8-pNCA unter Verwendung des "Molecule Builder" von SYBYL (Tripos, Inc., St. Louis, USA) erzeugt. Die F87A-Mutation wurde unter Verwendung des Biopolymer-Tools von SYBYL erzeugt. Die C-Atome 1 bis 4 des Substrats wurden in die Bindungsstelle entsprechend den C-Atomen 6 bis 9 der gebundenen Palmitoleinsäure plaziert. Die Torsionswinkel der Fettsäurekette wurden entsprechend der Trans-Konfiguration gewählt. NMR-Untersuchungen für P450 BM-3-Laurat- und -12-Bromlaurat-Komplexe zeigten, dass die Protonen der hydroxylierten C-Atome (C10 und C11) etwa 3,0 Å vom Häm-Eisen in der Oxidationsstufe II entfernt sind (10, 13). Auf der Basis dieses Ergebnisses wurden die entsprechenden Atome C7 und C8 von 8-pNCA in einem Abstand von 4 Å bzw. 3,6 Ä vom Häm-Eisen platziert. Die para-Nitrophenoxygruppe wurde manuell in der Bindungstasche angeordnet. Außerdem wurde die Energie des Komplexes über fixierte Backbone-Atome minimiert.

Methode 2: Sättigungsmutagenese

Die erfindungsgemäß verwendeten Mutanten wurden mit Hilfe der Sättigungsmutagenese unter Verwendung des Stratagene QuikChange Kit (La Jolla, Kalifornien, USA) hergestellt. Neun Positionen in der Umgebung des Substrat-Bindungskanals, und zwar P25, V26, R47, Y51, S72, A74, F87, L188 und M354, wurden für die Mutation über das P450 BM-3-Modeling ausgewählt. Die Primer für jede dieser Positionen sind in Tabelle 1 zusammengefasst.

Tabelle 1:

| ausgewählte Positionen | Primer | Sequenz Nr. |
|---|---|---|
| P25 | 5'-gttattaaacacagataaannngttcaagctttgatg-3'<br>5'-catcaaagcttgaacnnntttatctgtgtttaataac-3' | SEQ ID NO: 9<br>SEQ ID NO: 10 |
| V26 | 5'-gttattaaacacagataaaccgnnncaagctttgatg-3'<br>5'-catcaaagcttgnnncggtttatctgtgtttaataac-3' | SEQ ID NO: 11<br>SEQ ID NO: 12 |
| R47 | 5'-cgaggcgcctggtnnngtaacgcgctacttatc-3'<br>5'-gataagtagcgcgttacnnnaccaggcgcctcg-3' | SEQ ID NO: 13<br>SEQ ID NO: 14 |
| Y51 | 5'-cctggtcgtgtaacgcgcnnnttatcaagtcagc-3'<br>5'-gctgacttgataannngcgcgttacacgaccagg-3' | SEQ ID NO: 15<br>SEQ ID NO: 16 |
| S72 | 5'-gctttgataaaaacttannncaagcgcttaaatttgtacg-3'<br>5'-cgtacaaatttaagcgcttgnnntaagttttttatcaaagc-3' | SEQ ID NO: 17<br>SEQ ID NO: 18 |
| A74 | 5'-gctttgataaaaacttaaagtcaannncttaaatttgtacg-3'<br>5'-cgtacaaatttaagnnnttgaettaagttttttatcaaagc-3' | SEQ ID NO: 7<br>SEQ ID NO: 8 |
| L188 | 5'-gaagcaatgaacaagnnncagcgcgagcaaatccag-3'<br>5'-ctggatttgctcgetgnnncttgttcattgcttc-3' | SEQ ID NO: 5<br>SEQ ID NO: 6 |
| M354 | 5'-ggcgacgaactannngttctgattcctcag-3'<br>5'-ctgaggaatcagaacnnntagttcgtcgcc-3' | SEQ ID NO: 19<br>SEQ ID NO: 20 |
| F87 | 5'-gcaggagacgggggttgnnnacaagctggacg-3'<br>5'-cgtccagcttgtnnncaaccccgtctcctgc-3' | SEQ ID NO: 3<br>SEQ ID NO: 4 |

Die Reaktionsbedingungen waren für alle mutagenen PCR-Verfahren identisch, mit Ausnahme der Annealing-Temperatur, welche in folgender Weise variiert wurde: 50 °C für die Positionen 25, 26, 188 und 354; 52 °C für die Positionen 47 und 51; und 46 °C für die Positionen 72, 74 und 87. Die Reaktionen wurden in 50 $\mu$l Reaktionsvolumen durchgeführt, wobei jeder Ansatz 17,5 pmol jedes Primers, 20 pmol der Template-Plasmid-DNA, 3 U der Pfu-Polymerase und 3,25 nmol jedes dNTP enthielt. Die Reaktion wurde bei 95 °C, 4 Minuten, gestartet, anschließend wurde 20-mal folgender Thermocyclus durchlaufen: 95 °C, 1 Min.; 46-52 °C, 2,5 Min.; 72 °C, 17 Min.; nach diesen 20 Cyclen wurde die Reaktion 15 Minuten bei 72 °C fortgesetzt. Für die ortsspezifische Mutagenese durch PCR wurde ein Einzelcodon für den Austausch einer Aminosäure verändert. Für die Randomisierung einer spezifischen Aminosäure wurden Primer verwendet, in denen "nnn" für die spezifische Aminosäure, codiert. Alle PCR-Produktlösungen wurden mit 20 U DpnI bei 37 °C über 3 Stunden behandelt, um die ursprüngliche, nicht-mutierte Template-DNA zu verdauen. Anschließend erfolgte die Transformation in E. coli D85α.

Methode 3: Expression und Reinigung von Wildtyp-Enzym sowie der Mutanten

Das P450 BM-3-Wildtyp-Gen und dessen Mutanten wurden unter der Kontrolle des starken, Temperatur-induzierbaren $P_RP_L$-Promotors pCYTEXP1 in E. coli Stamm DH5α (supE44, lacU169 [80lacZ M15] hsdR17 recA1 endA1 gyrA96 thi-1 relA1) exprimiert. Die einzelne Punktmutation F87A wurde, wie aus dem Stand der Technik bekannt (12), eingeführt. Die transformierten Zellen wurden auf LB-Agarplatten ausplattiert, welche 100 μg/ml Ampicillin enthielten. Nach 12- bis 24-stündigem Wachstum wurden die Kolonien mit sterilen Zahnstochern aufgenommen und in Mikrotiterplatten mit 96 Vertiefungen plaziert, wobei jede Vertiefung 200 μl TB-Medium (12 g Tryptophan, 24 g Hefeextrakt, 4 ml Glyzerin, $H_2O$ (dest.) ad. 1 Liter) mit 100 μg/ml Ampicillin enthielt. Die Platten wurden über Nacht bei 37 °C inkubiert. Anschließend wurden 40 μl aus jeder Vertiefung entnommen und in Kulturröhrchen überführt, welche 2 ml TB-Medium mit 100 μg/ml Ampicillin enthielt, und anschließend wurde 2 Stunden bei 37 °C und dann 6 Stunden bei 42 °C inkubiert. Die Zellen wurden bei 4000 Upm 5 Minuten abzentrifugiert, mit Lysozym aus Hühnereiweiß (1 U/ml) behandelt und dann zweimal gefroren und aufgetaut. Die Zell-Rohextrakte erhielt man durch 10-minütige Zentrifugation bei 14000 Upm. Der so erhaltene Überstand wurde für die Aktivitätsmessung eingesetzt. Zur Herstellung großer Enzymmengen wurde ein 2 l-Schüttelkolben mit 300 ml TB-Medium mit 100 μg/ml Ampicillin verwendet, bei 37 °C inkubiert und 2 Stunden bei 200 Upm geschüttelt ($OD_{578nm}$ = 0,8 bis 1,0) und anschließend 6 Stunden bei 42 °C inkubiert. Die Zellen wurden durch 10-minütige Zentrifugation bei 4000 Upm gesammelt und in 15 ml 0,1 M Kaliumphosphat-Puffer, pH 7,4 suspendiert. Die eisgekühlte Suspension wurde mit Hilfe eines Branson Sonifiers W25 (Dietzenbach, Deutschland) (80 W, 2 Minuten, 3 Cyclen) aufgeschlossen. Die Suspension wurde 20 Minuten bei 32570 x g zentrifugiert. Die Rohextrakte wurden zur Bestimmung der Enzymaktivität oder zur Enzymreinigung verwendet.

Die Enzymreinigung wurde wie in (14) beschrieben, durchgeführt, wobei jedoch eine BioPilot-Chromatographieanlage (Pharmacia, Schweden) verwendet wurde. Die Reinheit des Enzyms wurde durch Bestimmung des Gesamtproteins sowie der Menge des Enzyms festgestellt. Die Konzentration an gereinigtem Enzym wurde über die Differenz zwischen dem Extinktionsspektrum des Carbonylkomplexes der Eisen-(II)-form im Vergleich zur Eisen-(II)-form unter Verwendung einer molekularen Absorptivität von 91 mM$^{-1}$ cm$^{-1}$ für das wellenlängenpaar 450 nm und 490 nm bestimmt (1).

Methode 4: Isolierung von Mutanten mit höherer Aktivität für Substrate kürzerer Kettenlänge

Die Mutante F87A von P450 BM-3 wurde anstelle des Wildtyps als Template-DNA verwendet. Die Mutationen für die jeweilige Position wurden wie oben beschrieben hergestellt. Jeweils etwa 100 Kolonien wurden zur Durchmusterung des Sequenzraumes an jeder Position gepickt, in Kulturröhrchen kultiviert und Zellen wurden daraus isoliert und lysiert. Die Zell-Rohextrakte einer jeden ausgewählten Kolonie wurden für den Aktivitätstest verwendet. Sämtliche Mutanten, die im Vergleich zu F87A eine höhere Aktivität für wenigstens ein Substrat mit einer kürzeren Kettenlänge als 15-pNCA zeigten, wurden zur Bestimmung der Mutationen sequenziert.

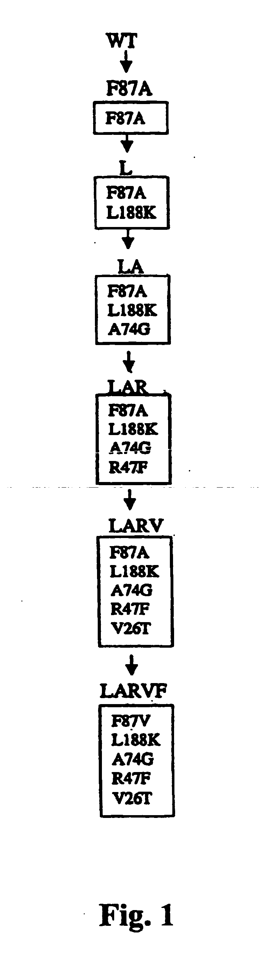

Die Mutante mit der jeweils höchsten Aktivität für 12-pNCA, 10-pNCA oder 8-pNCA von allen Mutanten für die gleiche Position wurde für eine spätere Kombination mit anderen Mutationen ausgewählt. Die kombinierte Mutation wurde schrittweise durch ortsspezifische Mutagenese ausgeführt. Der Kombinationsweg ist in Figur 1 dargestellt. Sechs Kolonien wurden für jeden Kombinationsschritt für die Bestimmung der Substratspezifität isoliert. Eine Kolonie mit repräsentativer Substratspezifität wurde ausgewählt und die Substratspezifität wurde für das reine Enzym bestimmt.

Das Plasmid der ausgewählten Kolonie wurde für den nächsten Schritt der ortsspezifischen Mutagenese verwendet. Die Mutationen in der Endmutante wurden durch DNA-Sequenzierung bestimmt (ABI PRISM$^R$ BigDye™ Terminator Cycle Sequencing Ready Reaction Kit und ABI Prism™ 377 DNA Sequencer).

Methode 5: Enzym-Aktivitätstest

Für den pNCA-Aktivitätstest wurden 8 μl einer 10 mM pNCA-Lösung in DMSO in einer Einwegküvette in einem Endvolumen von 1 ml verwendet. Nach Zugabe von 850 μl Tris/HCl-Puffer (0,1 M, pH 8,2) und 0,1 bis 0,4 nmol P450 zu der jeweiligen pNCA-DMSO-Lösung wurden die Proben 5 Minuten vorinkubiert, bevor die Reaktion durch Zugabe von 50 μl einer wässrigen Lösung von 1 mN NADPH gestartet wurde. Zur Bestimmung von Kcat und Km wurde eine Konzentrationsreihe für die verschiedenen pNCA-Substrate erstellt (bezüglich Details des Nachweisverfahrens kann auf (12) verwiesen werden).

Zur Durchführung des pNCA-Tests in einer Mikrotiterplatte wurde eine Platte mit 96 Vertiefungen (Greiner, Frickenhausen, Deutschland) verwendet. Der Reaktionsansatz enthielt in einem Gesamtreaktionsvolumen von 250 μl in Tris/HCl-Puffer (0,1 M, pH 8,2) entweder 60 nmol 8-pNCA, 15 nmol 10-pNCA, 15 nmol 12-pNCA oder 15 nmol 15-pNCA, jeweils gelöst in 2,5 μl DMSO. Nach 5-minütiger Vorinkubation mit 40 μl P450 BM-3-Proben wurde die Reaktion durch Injektion von 30 μl einer 1 mM NADPH-Lösung in jede Vertiefung gestartet. Die Platten wurden unmittelbar nach Zugabe der NADPH-Lösung auf einem Platten-Lesegerät bei 405 nm vermessen.

Methode 6: Umsetzung von Carbonsäuren mit erfindungsgemäßen Mutanten und Bestimmung der Produkte

a) Chemische Reaktion

Für die Hydroxylierung der Fettsäuren in Anwesenheit von P450 BM-3 bzw. seiner Mutanten wurde folgender Ansatz gewählt:

| | |
|---|---|
| P450 BM-3-Mutante | 20 mg |
| Reaktionspuffer | 20 ml (Tris/HCl 50mM, KCl 250mM, pH 7,8) |
| Fettsäure | 10 mg |
| Aceton | 400 μl (2 % v/v) (beschleunigt Reaktion um Faktor 2) |

Das Enzymlyophylisat wurde vor der Reaktion in 1 ml Reaktionspuffer gelöst und zunächst 30 min bei 36 °C inkubiert. Die Inkubation führt wie die Zugabe von 2 Vol.-% Aceton zu einer Aktivitätssteigerung von 60 bzw. 75 %.

Nach 5-minütiger Inkubation erfolgte die Zugabe von 500 μl der vorbereiteten NADPH-Lösung (12,5 mg/ml). Der Verlauf der Reaktion wurde durch Absorptionsmessungen bei 340 nm verfolgt, wobei der Verbrauch von NADPH beobachtet werden kann. Für eine stöchiometrische Umsetzung wären theoretisch 4 ml NADPH-Lösung notwendig, eine zu hohe Konzentration an NADPH in der Reaktionslösung führt jedoch zur Inaktivierung des Enzyms, deshalb wird der Cofaktor in 500 μl-Schritten zugegeben.

Nach Beendigung der Reaktion wurde die Lösung mit 5M Salzsäure bis zu einem pH-Wert von 2 angesäuert. Dabei fallen die Fettsäuren bzw. hydroxylierten Fettsäuren aus und führen zu einer sichtbaren Trübung der Lösung. Danach wurde zweimal mit je 10 ml Dichlormethan extrahiert und über Natriumsulfat getrocknet. Nach der Filtration über einen Faltenfilter erfolgte die Entfernung des Dichlormethans am Rotationsverdampfer oder durch Eindampfen mit Stickstoff. Der verbliebene weiße Feststoff wurde dann wieder in 2 ml Dichlormethan aufgenommen und für die GC-Analyse verwendet.

b) Gaschromatographische Analyse

Für die Analyse wurde ein Fisons Gaschromatograph (Fisons Instruments Mega Series, Mainz, Deutschland) mit FID verwendet (Optima 5-Säule, 25 m x 0,25 mm ID, Macherey & Nagel, Düren, Deutschland).

Zur Analyse wurden Edukte und Produkte mit MSHFBA silyliert. Dabei werden alle Hydroxyl- und Säuregruppen in die entsprechenden Trimethylsilylether bzw. -ester umgewandelt. Dabei ist darauf zu achten, dass die Probe wasserfrei ist, da es sonst zu Nebenreaktionen mit dem Silylierungsagens kommt. Zu 10 μl der hydroxyfettsäurehaltigen Dichlormethan-Lösung wurden 15 μl MSHFBA pipettiert und 15 min bei Raumtemperatur inkubiert. Nach Zugabe von 25 μl Dichlormethan erfolgte die GC-Analyse.

Dazu wurde 1 μl der silylierten Proben in den Gaschromatographen eingespritzt. Injektor- und Detektortemperatur lagen bei 350 °C. Folgende Temperaturprogramme wurden verwendet:

**Caprylsäure**

$$100 \text{ °C (2 min)} \xrightarrow{5\text{°/min}} 200 \text{ °C} \xrightarrow{10\text{°/min}} 300 \text{ °C (5 min)}$$

**Caprinsäure**

$$100 \ ^{\circ}C \ (2 \ min) \xrightarrow{5^{\circ}/min} 140 \ ^{\circ}C \ (5 \ min) \xrightarrow{10^{\circ}/min} 300 \ ^{\circ}C \ (5 \ min)$$

**Laurinsäure**

$$100 \ ^{\circ}C \ (2 \ min) \xrightarrow{5^{\circ}/min} 170 \ ^{\circ}C \ (5 \ min) \xrightarrow{10^{\circ}/min} 300 \ ^{\circ}C \ (5 \ min)$$

Beispiel 1: Selektion der Anfangsmutante

Die Einzelmutante F87A von P450 BM-3 wurde als Ausgangs-Template verwendet. Wie bereits erwähnt, wird durch diese Mutation die Hydroxylierungsposition in gesättigten C12- und C14-Fettsäuren von ω-1, ω-2 und ω-3 in Richtung der ω-Position verschoben (13). Für die Umsetzung von 12-pNCA und 15-pNCA wurde ebenfalls gezeigt, dass die ω-Hydroxylierung im Vergleich zum Wildtyp signifikant verstärkt wird (12). Für die ω-Hydroxylierung von 12-pNCA erhielt man eine vollständige Umwandlung, verglichen mit einer 33 %igen Umwandlung für den Wildtyp. Zusätzlich zur erhöhten Regioselektivität beobachtete man auch erhöhte Aktivitäten für 12-pNCA und 15-pNCA (vergleiche Tabelle 2).

Tabelle 2: Spezifische Aktivität ausgewählter P450 BM-3-Mutanten für verschiedene pNCA-Derivate mit unterschiedlicher Kettenlänge[1]

| Substrat | WT | F87A | L188K | V26T | R47F | S72G | A74G | M354T |
|---|---|---|---|---|---|---|---|---|
| 15-pNCA | 405 | 410 | 288 | 519 | 258 | 439 | 474 | 560 |
| 12-pNCA | 141 | 284 | 316 | 555 | 233 | 596 | 517 | 480 |
| 10-pNCA | 339 | 92 | 207 | 106 | 52 | 150 | 103 | 171 |
| 8-pNCA | 15 | 2 | 69 | 16 | 13 | 3 | 6 | 4 |
| [1] Einheiten spezifischer Aktivität: nmol/min/nmol P450 | | | | | | | | |

Die erhöhte Regioselektivität von F87A ist anhand des Strukturmodells verständlich. Die Substitution des sperrigen Restes F87 durch Alanin vergrößert die Bindungstasche für die p-Nitrophenoxygruppe, welche durch F87, außerdem durch V78, A82, T260, I263 und A264 gebildet wird. Außerdem wird der Zugang der großen p-Nitrophenoxygruppe zur Bindungstasche erleichtert und die sterischen Wechselwirkungen zwischen F87 und den C-Atomen ω-2 und ω-1 der pNCA werden eliminiert. Dies ermöglicht eine vorteilhaftere Orientierung der ω-Position gegenüber dem Häm-Eisenatom.

Beispiel 2: Selektion einzelner Mutationspositionen durch Modeling, ortsspezifische Randomisierungsmutagenese ausgewählter Positionen und Screening

1. Selektion der Mutationspositionen

Das Modell des gebundenen C8-pNCA-Substrats zeigt, dass sich das Carboxylat in einem Abstand von 9 und 11 Å zu den Carboxylat-bindenden Resten R47 bzw. Y51 befindet. Um Aktivität gegenüber C8-Substraten zu induzieren, ist es erforderlich, eine neue Bindungsstelle zu erzeugen, welche sich in geeigneter Distanz zur Carboxylatgruppe des Substrats befindet. Zur Erleichterung der Bindung von 8-pNCA sollten zusätzliche Reste innerhalb der Bindungsstelle positioniert werden. Folgende Reste wurden ausgewählt: Die Reste R47 und Y51, welche die ursprüngliche Carboxylat-Bindungsstelle bilden. Für R47 wird die Bildung eines Ionenpaares zwischen dessen Guanidiniumgruppe und dem Carboxylatrest des Substrats vorgeschlagen; Y51 bildet eine H-Brücke mit der Carboxylatgruppe des Substrats (9). Für den Palmitoleinsäure/P450 BM-3-Komplex beträgt die Distanz zwischen dem $C_\alpha$-Atom von R47 und dem C-Atom der Fettsäurecarboxylatgruppe 12 Å. Sämtliche Reste in der Hemisphäre mit einem 12 Å-Radius um die Carboxylatgruppe des 8-pNCA-Modells wurden bestimmt und nur solche innerhalb der Bindungstasche mit Seitenketten, die in Richtung der Carboxylatgruppe der Fettsäure weisen, wurden ausgewählt. So wurden P25, V26, S72, A74, L188 und M354 ausgewählt, welche wahrscheinlich eine neue Carboxylat-Bindungsstelle für kurzkettige pNCA-Verbindungen ausbilden könnten. Diese sechs ausgewählten Reste befinden sich auf sekundären Strukturelementen mit flexibler Konformation (11).

2. Mutation und Screening

Mutanten von jedem der acht ausgewählten Reste wurden durch ortsspezifische Randomisierung des Wildtyp-Codons an den entsprechenden Positionen erzeugt. Um sicherzustellen, dass die meisten der 19 möglichen Arten von Aminosäuren getestet werden, wurden 100 Kolonien einer jeden Position isoliert, kultiviert und auf Aktivität getestet. (Damit beträgt die Wahrscheinlichkeit, jede Aminosäure zu testen, mehr als 95 %, ausgenommen einer Wahrscheinlichkeit von 79 % für Tryptophan und Methionin). Die Mutanten mit einem höheren Aktivitätswert für wenigstens ein Substrat mit kürzerer Kettenlänge als 15-pNCA wurden für die Sequenzierung zur Bestimmung der Mutation (Mutationen) ausgewählt.

Es zeigte sich, dass Position 188 relativ variabel ist. Die meisten der 100 Kolonien zeigten Aktivität für pNCA. Daraus wurden entsprechend ihrer Aktivität gegenüber 8-pNCA 37 Kolonien ausgewählt. 16 verschiedene Aminosäuretypen, einschließlich dem Wildtyp wurden detektiert. Dieses Ergebnis bestätigte außerdem, dass die Auswahl von 100 Kolonien ausreichend war, um sicherzustellen, dass die meisten der 20 möglichen Aminosäuren getestet werden können. Von den 15 Substitutionen erhöhten die Substitutionen K, R, W, Q, N, G, A und S signifikant die katalytische Aktivität für Substrate mit kürzerer Kettenlänge. Die Substitution von L durch negativ geladene Aminosäuren führte zu einer Verringerung der Aktivität gegenüber 10-pNCA, 12-pNCA und 15-pNCA um das Drei- bis Siebenfache.

Die anderen sieben Positionen wurden in ähnlicher Weise wie Position 188 randomisiert. Von jeder Position wurden 7 bis 19 Kolonien für die DNA-Sequenzierung zum Nachweis der Mutation (Mutationen) ausgewählt. Die meisten dieser Gene waren entweder nicht mutiert oder das Genprodukt zeigte eine verringerte Aktivität für Substrate kürzerer Kettenlänge (Tests wurden mit reinem Enzym durchgeführt). Jeweils nur eine Mutante der Positionen 26, 47, 74 und 354, zwei Mutanten der Position 72 und keine Mutante der Positionen P25 und Y51 zeigte im Vergleich zu 15-pNCA für Substrate kürzerer Kettenlänge eine höhere Aktivität. Die Mutante mit der höchsten Aktivität für 8-pNCA in Position 188 und Position 72 sowie sämtliche Einzelmutanten der Positionen 26, 47, 74 und 354 wurden für eine Kombination ausgewählt. Diese Mutationen, welche in obiger Tabelle 2 aufgelistet sind, enthalten folgende Substitutionen: V26T, R47F, S72G, A74G, L188K und M354T. Im Vergleich zu F87A erhöhte sich die spezifische Aktivität gegenüber 8-pNCA um den Faktor 0,5 bis 33,5. Jede dieser Mutationen wurde mit der Ursprungsmutation F87A schrittweise kombiniert.

Beispiel 3: mehrfachmutanten, hergestellt durch schrittweise Kombination von Mutanten und deren Screening

1. Mutante L (F87A, L188K)

Die Auswahl der ersten Mutante ist kritisch, da die Kombination von Einzelmutanten nicht notwendigerweise in einer Kumulation der Einzeleffekte resultiert. Eine falsche Auswahl der ersten Mutation könnte in die Richtung eines Evolutionsweges lenken, der nicht zu einer optimalen Mehrfachmutation führt. Im Falle eines großen Ausgangspools von Einzelmutanten ist daher eine enorme kombinatorische Vielfalt wahrscheinlich, und das Auffinden der gewünschten Mehrfachmutante ist folglich unwahrscheinlich.

zwei Kriterien sprechen für die Auswahl der Mutante L als Ausgangspunkt: deren erhöhte Aktivität gegenüber 8-pNCA im Vergleich zu allen anderen Mutanten (Tabelle 2) sowie die Veränderungen in der Enzym-Substratwechselwirkung und den Eigenschaften der Bindungsstelle. Zur Verifizierung dieser Veränderungen wurden Strukturmodelle der ausgewählten Mutanten dadurch erzeugt, dass man die Seitenketten der Mutationspositionen mit Hilfe der SYBYL-Methode ersetzte. L188 ist am C-Terminus der α-Helix F lokalisiert. Diese Helix und die benachbarten G Helices erfahren die größte Verschiebung durch eine Substratbindung (9). Das Modell veranschaulicht, dass ein Austausch von Leucin durch Lysin zur Bildung einer neuen putativen Carboxylat-Bindungsstelle führt. Der Abstand zwischen der Aminogruppe von K188 und dem Carboxylatrest von 8-pNCA beträgt 6 Å und ist somit vergleichbar mit dem experimentell bestimmten Abstandswert für die Carboxylatgruppe der Palmitoleinsäure und der Guanidiniumgruppe von R47 (dem ursprünglichen Bindungsrest). Die Distanz zwischen der Guanidiniumgruppe von R47 und der Carboxylatgruppe von 8-pNCA beträgt 11,4 Å. Es kann daher vorgeschlagen werden, dass die Ionenpaar-Wechselwirkungen zwischen der Carboxylatgruppe des Substrates und K188 zusammen mit dem hydroxylierten C8-Atom in reaktiver Distanz zum Häm-Eisenatom erfolgen.

Die anderen fünf Mutanten wurden schrittweise mit der Mutante L durch ortsspezifische Mutagenese kombiniert (vergleiche Figur 1). Für jeden Mutagenese-Schritt wurde ein Strukturmodell der selektierten Mutanten erzeugt. Anhand dieses Modells wurden die Einflüsse der erzeugten Mutanten auf die Substratbindung untersucht und eine rationale Erklärung für die optimierten Eigenschaften der Mutanten und ihrer Kombinationen wurde davon ausgehend vorgeschlagen.

2. Mutante LA (F87A, L188K, A74G)

Die katalytische Effektivität gegenüber 8-pNCA erhöhte sich durch Einführung der Mutation A74G in die Mutante L. A74 befindet sich am N-Terminus der $\alpha$-Helix B'. Da die Seitenkette von A74 sterisch mit der Aminogruppe von K188 interagiert, wird durch einen Austausch von A74 durch Glycin diese Wechselwirkung eliminiert, wodurch eine vorteilhaftere Orientierung der Seitenkette gegenüber der Carboxylatgruppe des Substrats ermöglicht wird.

3. Mutante LAR (F87A, L188K, A74G, R47F)

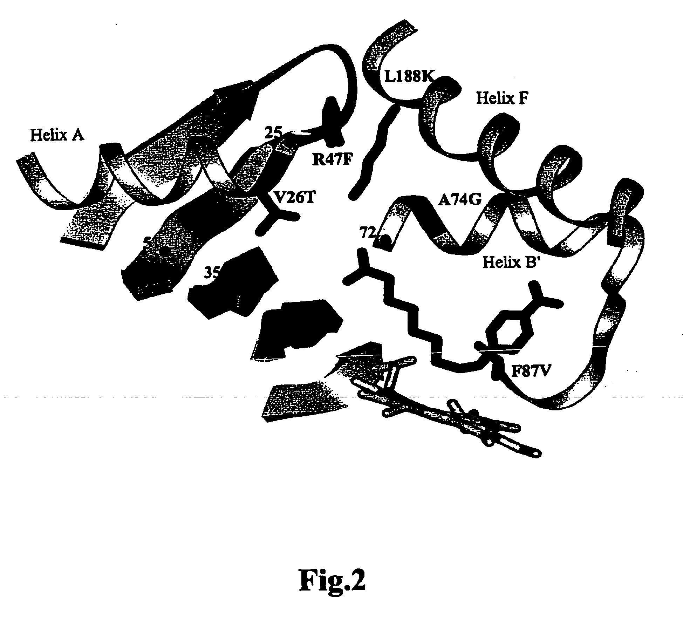

Die Verbesserung der katalytischen Effizienz für 8-pNCA gelang durch Einführung der zusätzlichen Mutation R47F. Die Mutation R47F hat zwei mögliche Effekte. Phenylalanin behindert die ursprüngliche Carboxylatbindung und vergrößert den dem Lösungsmittel ausgesetzten hydrophoben Abschnitt am Eingang des Bindungskanals, der von den Resten F11, L14, L17, P18, P45 und A191 gebildet wird (vergleiche Figur 2). Dieser hydrophobe Abschnitt ist von besonderer Bedeutung für die Substratanbindung (11).

4. Mutante LARV (F87A, L188K, A74G, R47F, V26T)

Wird die Mutation M354T der Mutante LAR hinzugefügt, so verringert sich Kcat für 8-pNCA. M354T wurde deshalb aus weiteren Kombinations-Versuchen ausgeklammert. Fügt man jedoch die Mutation V26T der Mutante LAR zu, so zeigt die resultierende Mutante LARV einen geringfügig höheren Kcat-Wert und einen geringfügig geringeren Km-Wert als LAR. Die katalytische Effizienz gegenüber 8-pNCA erhöhte sich. Wird statt dessen die Mutation S72G in der Mutante LAR durchgeführt, so verringerte sich Kcat für 8-pNCA. Aus diesem Grund wurde die Mutante LARV für weitere Mutationen ausgewählt.

V26 ist am N-Terminus der $\alpha$-Helix A lokalisiert. Das Modell weist darauf hin, dass T26 die Rolle des ursprünglichen Restes Y51 der Bindungsstelle übernehmen könnte und die Carboxylatgruppe des Substrates durch Ausbildung einer H-Brücke stabilisieren könnte. Der Abstand zwischen der Hydroxylgruppe von T26 und der Aminogruppe von K188 beträgt 6,9 Å. Verglichen mit der ursprünglichen Bindungsstelle ist dieser Abstand um etwa 2 Å größer (4,8 Å zwischen der Hydroxygruppe von Y51 und der Guanidiniumgruppe von R47). Es besteht somit die Möglichkeit, dass die K188-haltige F-Helix weiteren Konformations-Änderungen unterzogen wird, aufgrund derer 8-pNCA tiefer in der Bindungsregion festgehalten wird und die Distanz zwischen T26 und K188 verringert wird.

5. Mutante LARVF (F87V, L188K, A74G, R47F, V26T)

Es konnte gezeigt werden, dass Position 87 von besonderer Bedeutung für die katalytische Aktivität und die Substratspezifität des Enzyms ist (3, 13). Die Mutante LARV wurde deshalb in Position 87 durch ortsspezifische randomisierte Mutagenese optimiert. Von 100 Kolonien wurde eine Mutante erhalten, welche eine katalytische Effizienz von $3,5 \times 10^4$ s$^{-1}$ M$^{-1}$ gegenüber 8-pNCA zeigte. Die DNA-Sequenzdaten ergaben, dass Alanin in Position 87 durch Valin ersetzt war.

Aus der Kristallstruktur (11) und früheren Experimenten (3, 13) ist bekannt, dass R87 wichtig für den Zugang des Substrates zum Häm-Eisen ist. Die sperrige para-Nitrophenylgruppe dieses Substrats machte es notwendig, die Größe der Bindungsstelle durch den Austausch von Phenylalanin durch Alanin zu erhöhen. Durch den Austausch von A87 durch Valin wird der Kontakt von $C_\omega$ mit dem Häm-Eisenatom aufgrund sterischer Wechselwirkungen zwischen dem Ethersauerstoff und der Seitenkette von V87 verbessert.

Die Daten zeigen,-dass- zwei Schlüsselschritte, F87A zu L und LARV zu LARVF, die katalytische Effizienz gegenüber 8-pNCA erhöhten. Es stellt sich deshalb die Frage, ob die anderen Mutationen in der Mutante LARVF wieder rückgängig gemacht werden können, ohne die Aktivität für 8-pNCA zu verlieren. Deshalb wurden Mutanten hergestellt, die statt der Mutation F87A die Mutation F87V enthalten. Diese Mutanten tragen folgende Bezeichnungen:

1.) L7V mit den Mutationen F87V L188K
2.) AL7V mit den Mutationen F87V L188K A74G
3.) ARL7V mit den Mutationen F87V L188K A74G R47F

Bei den Aktivitätstests wurden folgende Ergebnisse erhalten:

Tabelle 3: Kinetische Parameter von P450 BM-3-Mutanten für pNCA-Derivate unterschiedlicher Kettenlänge, ermittelt bei pH 8,0 und 25 °C

| | Kcat ($s^{-1}$) | | | Km ($\mu M$) | | | Kcat/Km ($s^{-1}M^{-1}$) | | |
|---|---|---|---|---|---|---|---|---|---|
| | 12 | 10 | 8 | 12 | 10 | 8 | 12 | 10 | 8 |
| F87V | 0,7 | 1,8 | - | 3,8 | 8,5 | - | $1,7 \cdot 10^5$ | $2,1 \cdot 10^5$ | - |
| L7V | 2,8 | 4,7 | - | 6,4 | 14,2 | - | $4,3 \cdot 10^5$ | $3,3 \cdot 10^5$ | - |
| AL7V | 2,2 | 5,9 | 4,3 | 15,1 | 22,7 | 197,6 | $1,4 \cdot 10^5$ | $2,6 \cdot 10^5$ | $2,0 \cdot 10^4$ |
| ARL7V | 1,4 | 5,5 | 3,9 | 8,9 | 17,5 | 41,4 | $1,7 \cdot 10^5$ | $3,1 \cdot 10^5$ | $9,3 \cdot 10^4$ |
| ARVLF | 1,4 | 7,2 | 0,2 | 12,3 | 44,8 | 6,5 | $9,2 \cdot 10^4$ | $1,6 \cdot 10^5$ | $3,5 \cdot 10^4$ |

Die Ergebnisse zeigen, dass die Mutanten F87V und L7V für das Substrat 8-pNCA keinen messbaren Umsatz zeigen. Die Ergebnisse zeigen weiterhin, dass die Vierfachmutante ARL7V eine noch bessere katalytische Effizienz aufweist als die Fünffachmutante ARVLF.

Die Mutanten ARL7V und ARVLF zeigen weiterhin hohe Aktivitäten für Caprinsäure (C10), die um zwei Kohlenstoffatome kürzer ist als das native kürzeste Fettsäure-Substrat des Wildtyp-Enzyms. Außerdem wurden ω-4-monohydroxylierte Produkte beobachtet, wenn Laurinsäure (C12) als Substrat verwendet wird, wohingegen das Wildtyp-Enzym von P450 BM-3 nur in den Positionen ω-1, ω-2 und ω-3 aktiv ist.

Beispiel 4: Bestimmung der bevorzugten Hydroxylierungsposition für Carbonsäuren unterschiedlicher Kettenlänge und verschiedene Mutanten und Vergleich mit dem Wildtyp-Enzym

Die Reaktionsansätze werden gemäß oben beschriebener Methode 6 aufgearbeitet und analysiert.
Die Ergebnisse sind in folgender Tabelle 4 zusammengefasst.

Tabelle 4:

| Hydroxylierungspositionen | Caprinsäure (C10) [%] | Laurinsäure (C12) [%] |
|---|---|---|
| Wildtyp | | |
| ω-3 | - | 34 |
| ω-2 | - | 28 |
| ω-1 | - | 38 |
| Mutante L7V | | |
| ω-3 | - | 53 |
| ω-2 | - | 30 |
| ω-1 | - | 17 |
| Mutante F87V | | |
| ω-3 | - | 51 |
| ω-2 | - | 25 |
| ω-1 | - | 24 |
| Mutante AL7V | | |
| ω-3 | - | 28 |
| ω-2 | - | 54 |
| ω-1 | - | 18 |
| Mutante ARL7V | | |

(fortgesetzt)

| Hydroxylierungspositionen | Caprinsäure (C10) [%] | Laurinsäure (C12) [%] |
|---|---|---|
| ω-3 | 14 | 35 |
| ω-2 | 33 | 50 |
| ω-1 | 53 | 15 |
| Mutante ARVLF | | |
| ω-3 | 15 | 34 |
| ω-2 | 30 | 53 |
| ω-1 | 55 | 13 |

Auffallend bei der Hydroxylierung von Laurinsäure ist zunächst ein Wechsel der Regioselektivität beim Übergang vom Wildtyp zu P450 BM-3 F87V, das eine ω-3-Hydroxylierung bevorzugt katalysiert, ebenso wie P450 BM-3 L7V. Beim Übergang zur Dreifachmutante ändert sich die Regioselektivität erneut, P450 BM-3 AL7V, P450 BM-3 ARL7V und P450 BM-3 ARVLF dirigieren die Hydroxylierung bevorzugt in die ω-2-Position.

Alle GC-Analysen zeigen, dass Caprinsäure nur von P450 BM-3 ARVLF und P450 BM-3 ARL7V umgesetzt wird, bei den anderen Mutanten wurde ein Umsatz unter 1 % festgestellt. Beide Enzyme weisen fast identische Regioselektivität, mit der Bevorzugung ω-1-Position auf.

Zur Bestimmung der Reaktionsausbeuten wurden Gaschromatogramme von Standards aufgenommen. Als Eduktstandard fand eine Caprinsäure- bzw. Laurinsäurelösung mit Dichlormethan als Lösungsmittel (Konzentration 0,5 mg/ml) Verwendung. Aufgrund der FID-Detektionsmethode können die Peakflächen von Edukt und Produkten nicht einfach zueinander ins Verhältnis gesetzt werden, da Moleküle unterschiedlicher Struktur- und Summenformel bei der Verbrennung unterschiedliche Ionenströme erzeugen. Diese Ionenströme sind nicht proportional zum Stoffmengenverhältnis von Edukt und Produkten. Als Produktstandard wurde deshalb käuflich erwerbbare 10-Hydroxycaprinsäure bzw. 12-Hydroxylaurinsäure verwendet. Die Summenformeln der Standards und der Produkte sind gleich, und die Struktur unterscheidet sich nur in der stellung der Hydroxylgruppe, weshalb bei gleichen Stoffmengen annähernd von gleichen detektierbaren Ionenströmen ausgegangen wird.

Bei den Hydroxylierungen von Caprinsäure mit P450 BM-3 ARVLF und P450 BM-3 ARL7V-Katalyse betrugen die kumulierten Ausbeuten an Produkt 57 % bzw. 38 %. Bei den Hydroxylierungen von Laurinsäure betrug die Ausbeute bei P450 BM-3 ARVLF-Katalyse 51 %, bei allen anderen Mutanten und dem Wildtyp zwischen 38 % und 40 %.

Literatur

1. Boddupalli, S. S., et al., (1990) J. Biol. Chem. 265, 4233-4239

2. Capdevila, J. H., et al., (1996) J. Biol. Chem. 271, 22663-22671

3. Graham-Lorence, S., et al., (1997) J. Biol. Chem. 272, 1127-1135

4. Cleland, J. L. und Craik, C. S., Eds. (1996) Protein Engineering: Principles and Practice, Wiley-Liss, New York

5. Kuchner, 0. und Arnold, F. H., (1997) Trends Biotechnol. 15, 523-530

6. Stemmer, W. P.C. (1994) Nature (London) 370,389-391

7. Bornscheuer, U. T., (1998) Angew. Chem 110, 3285-3288

8. Bernstein, F. C., et al.,(1977) J. Mol. Biol. 112, 525-542

9. Li, H. und Poulos, T. L. (1997) Nat. Structural Biol. 4, 140-146

10. Modi, S., et al.,(1996) Nat. Structural Biol. 3, 414-417

11. Ravichandran, K. G., et al., (1993) Science 261, 731-736

12. Schwaneberg, U., et al., (1999) Anal. Biochem. 269, 359-366

13. Oliver, C.F., et al.,(1997) Biochemistry, 36, 1567-1572

14. Schwaneberg, U., et al., J. Chromatography A, in press

15. Cherry, J. R., et al., (1999) Nature Biotechnology, 17, 379-384

## Patentansprüche

1.  Modifizierte Cytochrom P450 Monooxygenase, welche durch ortsspezifische Mutagenese ihres Substrat-bindenden Bereichs im Vergleich zum Wildtyp ein verändertes Substratprofil bei der terminalen und/oder subterminalen enzymatischen Hydroxylierung von aliphatischen Carbonsäuren zeigt; abgeleitet von Cytochrom P450 Monooxygenase BM-3 aus Bacillus megaterium mit einer Aminosäuresequenz gemäß SEQ ID NO:2, enthaltend wenigstens je eine funktionale Mutation in den Aminosäuresequenzpositionen 87 und 188 und gegebenenfalls wenigstens eine funktionale Mutation in einem der folgenden Aminosäuresequenzpositionen: 26, 47, 72, 74, und 354; wobei

Phe 87 ersetzt ist durch Val, Ala oder Leu;
Leu 188 ersetzt ist durch Asn, Gln, Arg, Lys, Ala, Gly, Ser oder Trp;
Ala 74 ersetzt ist durch Val oder Gly;
Arg 47 ersetzt ist durch His Tyr oder Phe;
Val 26 ersetzt ist durch Ser oder Thr,
Ser 72 ersetzt ist durch Ala, Val, Leu, Ile oder Gly; oder
Met 354 ersetzt ist durch Ser oder Thr.

2.  Monooxygenase nach Anspruch 1, **dadurch gekennzeichnet, dass** sie wenigstens eines der folgenden Aminosäuresubstitutionsmuster aufweist:

    b) F87A L188K;
    c) F87V L188K;
    d) F87A L188K A74G;
    e) F87V L188K A74G;
    f) F87A L188K A74G R47F;
    g) F87VL188K A74G R47F;
    h) F87A L188K A74G R47F V26T; oder
    i) F87V L188K A74G R47F V26T.

3.  Nukleinsäuresequenz, kodierend für eine Monooxygenase nach einem der vorherigen Ansprüche und deren komplementäre Nukleinsäuresequenz.

4.  Expressionskonstrukt, enthaltend unter der genetischen Kontrolle regulativer Nukleinsäuresequenzen eine kodierende Sequenz, welche eine Nukleinsäuresequenz nach Anspruch 3 umfasst.

5.  Vektor, umfassend wenigstens ein Expressionskonstrukt nach Anspruch 4.

6.  Rekombinanter Mikroorganismus, enthaltend mit wenigstens einem Vektor nach Anspruch 5.

7.  Mikroorganismus nach Anspruch 6, ausgewählt unter Bakterien der Gattung Escherichia.

8.  Verfahren zur enzymatischen Herstellung von terminal oder subterminal hydroxylierten aliphatischen Carbonsäuren, **dadurch gekennzeichnet, dass** man

    a1) einen rekombinanten Mikroorganismus nach Anspruch 6 oder 7 in Gegenwart eines Kulturmediums, enthaltend wenigstens eine hydroxylierbare Carbonsäure oder wenigstens ein hydroxylierbares Carbonsäurederivat, kultiviert; oder
    a2) ein Reaktionsmedium, enthaltend wenigstens eine hydroxylierbare Carbonsäure oder wenigstens ein hydroxylierbares Carbonsäurederivat, mit einem Enzym nach einem der Ansprüche 1 und 2 inkubiert; und
    b) das gebildete hydroxylierte Produkt aus dem Medium isoliert.

9.  Verfahren nach Anspruch 8, **dadurch gekennzeichnet, dass** man als hydroxylierbare Carbonsäure eine $C_8$-$C_{12}$-Monocarbonsäure oder ein Derivat davon einsetzt.

10. Verfahren nach einem der Ansprüche 8 und 9, **dadurch gekennzeichnet, dass** man die Reaktion in Gegenwart eines Elektronendonors oder Reduktionsäquivalents durchführt.

11. Verfahren nach Anspruch 10, **dadurch gekennzeichnet, dass** der Elektronendonor oder das Reduktionsäquivalent ausgewählt ist unter NADH, NADPH und Zn/Co(III)sepulchrat.

**Claims**

1.  A modified cytochrome P450 monooxygenase which, in comparison with the wild-type enzyme, shows an altered substrate profile in the terminal and/or subterminal enzymatic hydroxylation of aliphatic carboxylic acids, owing to site-specific mutagenesis of its substrate-binding region; derived from Bacillus megaterium cytochrome P450 monooxygenase BM-3 with an amino acid sequence in accordance with SEQ ID NO: 2, comprising at least in each case one functional mutation in the amino acid sequence positions: 87 and 188 and optionally at least one functional mutation in one of the following amino acid sequence positions 26, 47, 72, 74 and 354; where

Phe 87 is replaced by Val, Ala or Leu;
Leu 188 is replaced by Asn, Gln, Arg, Lys, Ala, Gly, Ser or Trp;
Ala 74 is replaced by Val or Gly;
Arg 47 is replaced by His, Tyr or Phe;
Val 26 is replaced by Ser or Thr;
Ser 72 is replaced by Ala, Val, Leu, Ile or Gly; or
Met 354 is replaced by Ser or Thr.

2. The monooxygenase according to claim 1, which comprises at least one of the following amino acid substitution patterns:

    b) F87A L188K;
    c) F87V L188K;
    d) F87A L188K A74G;
    e) F87V L188K A74G;
    f) F87A L 188K A74G R47F;
    g) F87V L188K A74G R47F;
    h) F87A L188K A74G R47F V26T; or
    i) F87V L188K A74G R47F V26T.

3. A nucleic acid sequence encoding a monooxygenase according to any of the preceding claims and the complementary nucleic acid sequence thereof.

4. An expression construct comprising, under the genetic control of regulatory nucleic acid sequences, an encoding sequence which comprises a nucleic acid sequence according to claim 3.

5. A vector which comprises at least one expression construct according to claim 4.

6. A recombinant microorganism containing at least one vector according to claim 5.

7. The microorganism according to claim 6, selected from among bacteria of the genus Escherichia.

8. A process for the enzymatic production of terminally or subterminally hydroxylated aliphatic carboxylic acids, which comprises

a1) culturing a recombinant microorganism according to claim 6 or 7 in the presence of a culture medium which comprises the at least one hydroxylatable carboxylic acid or at least one hydroxylatable carboxylic acid derivative; or
a2) incubating a reaction medium comprising at least one hydroxylatable carboxylic acid or at least one hydroxylatable carboxylic acid derivative with an enzyme according to either of claims 1 and 2, and
b) isolating the resulting hydroxylated product from the medium.

9. The process according to claim 8, wherein the hydroxylatable carboxylic acid is a $C_8$-$C_{12}$ monocarboxylic acid or a derivative thereof.

10. The process according to either of claims 8 and 9, wherein the reaction is carried out in the presence of an electron donor or a reduction equivalent.

11. The process according to claim 10, wherein the electron donor or the reduction equivalent is selected from amongst NADH, NADPH and Zn/Co(III) sepulchrate.

**Revendications**

1. Mono-oxygénase de cytochrome P450 modifiée, qui présente par mutagenèse spécifique au site dans sa région de liaison au substrat, en comparaison du type sauvage, un profil de substrat modifié en matière d'hydroxylation enzymatique terminale et/ou subterminale d'acides carboxyliques aliphatiques; dérivée de la mono-oxygénase de cytochrome P450 BM-3 provenant de l'espèce *Bacillus megaterium* ayant une séquence d'acides aminés représentée par la SEQ ID n° 2, contenant au moins, respectivement, une mutation fonctionnelle au niveau des positions de séquence d'acides aminés 87 et 188 et, éventuellement, au moins une mutation fonctionnelle à l'une des positions de séquence d'acides aminés suivantes : 26, 47, 72, 74 et 354; dans laquelle :

- le résidu Phe 87 est remplacé par un acide aminé de type Val, Ala ou Leu;
- le résidu Leu 188 est remplacé par un acide aminé de type Asn, Gln, Arg, Lys, Ala, Gly, Ser ou Trp;
- le résidu Ala 74 est , remplacé par un acide aminé de type Val ou Gly;
- le résidu Arg 47 est remplacé par un acide aminé de type His, Tyr ou Phe;
- le résidu Val 26 est remplacé par un acide aminé de type Ser ou Thr;
- le résidu Ser 72 est remplacé par un acide aminé de type Ala, Val, Leu, Ile ou Gly; ou
- le résidu Met 354 est remplacé par un acide aminé de type Ser ou Thr.

2. Mono-oxygénase selon la revendication 1, **caractérisée en ce qu'**elle présente au moins un des profils de substitution d'acides aminés suivants:

b) F87A, L188K;
c) F87V, L188K;
d) F87A, L188K, A74G;
e) F87V, L188K, A74G;
f) F87A, L188K, A74G, R47F;
g) F87V, L188K, A74G, R47F;
h) F87A, L188K, A74G, R47F, V26T; ou
i) F87V, L188K, A74G, R47F, V26T.

3. Séquence d'acide nucléique, codant pour une mono-oxygénase selon l'une quelconque des revendications précédentes, et sa séquence d'acide nucléique complémentaire.

4. Construction d'expression, contenant sous le contrôle génétique de séquences d'acides nucléiques de régulation, une séquence codante qui comprend une séquence d'acide nucléique selon la revendication 3.

5. Vecteur, comprenant au moins une construction d'expression selon la revendication 4.

6. Micro-organisme recombinant, contenant au moins un vecteur selon la revendication 5.

7. Micro-organisme selon la revendication 6, choisi parmi les bactéries du genre *Escherichia.*

8. Procédé de fabrication enzymatique d'acides carboxyliques aliphatiques hydroxylés en position terminale ou subterminale, **caractérisé en ce que** :

a1) on cultive un micro-organisme recombinant selon la revendication 6 ou 7, en présence d'un milieu de culture qui contient au moins un acide carboxylique pouvant être hydroxylé ou au moins un dérivé d'acide carboxylique pouvant être hydroxylé; ou
a2) on incube un milieu réactionnel contenant au moins un acide carboxylique pouvant être hydroxylé ou au moins un dérivé d'acide carboxylique pouvant être hydroxylé, avec une enzyme selon l'une quelconque des revendications 1 et 2; et
b) on isole le produit hydroxylé formé du milieu.

9. Procédé selon la revendication 8, **caractérisé en ce que** l'on utilise, comme acide carboxylique pouvant être hydroxylé, un acide monocarboxylique en $C_8$-$C_{12}$ ou un dérivé de celui-ci.

10. Procédé selon l'une quelconque des revendications 8 et 9, **caractérisé en ce que** l'on effectue la réaction en présence d'un donneur d'électrons ou d'un équivalent de réduction.

11. Procédé selon la revendication 10, **caractérisé en ce que** le donneur d'électrons ou l'équivalent de réduction est choisi parmi le NADH, le NADPH et le système Zn/sépulcrate de Co(III).

WT
↓

F87A

| F87A |
|------|

↓

L

| F87A<br>L188K |
|------|

↓

LA

| F87A<br>L188K<br>A74G |
|------|

↓

LAR

| F87A<br>L188K<br>A74G<br>R47F |
|------|

↓

LARV

| F87A<br>L188K<br>A74G<br>R47F<br>V26T |
|------|

↓

LARVF

| F87V<br>L188K<br>A74G<br>R47F<br>V26T |
|------|

## Fig. 1

**Fig.2**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 19935115 A

### In der Beschreibung aufgeführte Nicht-Patentliteratur

- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- **POUWELS P. H. et al.** Cloning Vectors. Elsevier, 1985
- **F. AUSUBEL et al.** Current Protocols in Molecular Biology. Wiley Interscience, 1997
- **BODDUPALLI, S. S. et al.** *J. Biol. Chem.,* 1990, vol. 265, 4233-4239
- **CAPDEVILA, J. H. et al.** *J. Biol. Chem.,* 1996, vol. 271, 22663-22671
- **GRAHAM-LORENCE, S. et al.** *J. Biol. Chem.,* 1997, vol. 272, 1127-1135
- Protein Engineering: Principles and Practice. Wiley-Liss, 1996
- **KUCHNER, 0. ; ARNOLD, F. H.** *Trends Biotechnol.,* 1997, vol. 15, 523-530
- **STEMMER, W. P.C.** *Nature,* 1994, vol. 370, 389-391
- **BORNSCHEUER, U. T.** *Angew. Chem,* 1998, vol. 110, 3285-3288
- **BERNSTEIN, F. C. et al.** *J. Mol. Biol.,* 1977, vol. 112, 525-542
- **LI, H. ; POULOS, T. L.** *Nat. Structural Biol.,* 1997, vol. 4, 140-146
- **MODI, S. et al.** *Nat. Structural Biol.,* 1996, vol. 3, 414-417
- **RAVICHANDRAN, K. G. et al.** *Science,* 1993, vol. 261, 731-736
- **SCHWANEBERG, U. et al.** *Anal. Biochem.,* 1999, vol. 269, 359-366
- **OLIVER, C.F. et al.** *Biochemistry,* 1997, vol. 36, 1567-1572
- **SCHWANEBERG, U. et al.** *J. Chromatography A*
- **CHERRY, J. R. et al.** *Nature Biotechnology,* 1999, vol. 17, 379-384